# EUROPEAN PATENT SPECIFICATION

(11) **EP 1 017 803 B1**
(45) Date of publication and mention of the grant of the patent: **16.06.2010**
(21) Application number: 98949508.0
(22) Date of filing: 25.09.1998
(51) Int. Cl.: C12N 15/11, C12N 15/63, C12N 15/85, C12N 15/86, C12N 15/00, C12P 21/00, A61K 48/00

(54) **EXPRESSION OF ENDOGENOUS GENES BY NON-HOMOLOGOUS RECOMBINATION OF A VECTOR CONSTRUCT WITH CELLULAR DNA**
EXPRESSION VON ENDOGENEN GENEN DURCH HOMOLOGE REKOMBINATION EINES VEKTORKONSTRUKTES MIT ZELLULÄRER DNA
EXPRESSION DE GENES ENDOGENES PAR RECOMBINAISON NON HOMOLOGUE D'UNE CONSTRUCTION VECTORIELLE A L'AIDE D'ADN CELLULAIRE

(30) Priority: 26.09.1997 US 941223; 24.09.1998 US 159643
(43) Date of publication of application: 12.07.2000
(73) Proprietor: ABT Holding Company, Cleveland, OH 44115-2634 (US)
(72) Inventor: HARRINGTON, John, J., Mentor, OH 44060-3052 (US)
(74) Representative: Wright, Simon Mark
(86) International application number: PCT/US1998/020094
(87) International publication number: WO 1999/015650

(56) References cited:
- WO-A1-95/31560
- US-A- 5 024 939
- US-A- 5 561 053
- US-A- 5 578 461
- US-A- 5 641 670

### SEQUENCE LISTING

<110> Harrington, John J. Athersys, Inc.
<120> Expression of Endogenous Genes by Non-homologous Recombination of a Vector Construct With Cellular DNA
<130> 1522.003PC01
<140> To be assigned
   <141> 1998-09-25
<150> TO BE ASSIGNED
   <151> 1998-09-24
<150> 08/941,223
   <151> 1997-09-26
<160> 13
<170> PatentIn Ver. 2.0
<210> 1
   <211> 39
   <212> DNA
   <213> Homo sapiens
<400> 1
   tccttcgaag cttgtcatgg ttggttcgct aaactgcat 39
<210> 2
   <211> 40
   <212> DNA
   <213> Homo sapiens
<400> 2
   aaacttaaga tcgattaatc attcttctca tatacttcaa 40
<210> 3
   <211> 28
   <212> DNA
   <213> Homo sapiens
<400> 3
   atccaccatg gctacaggtg agtactcg 28
<210> 4
   <211> 36
   <212> DNA
   <213> Homo sapiens
<400> 4
   gatccgagta ctcacctgta gccatggtgg atttaa 36
<210> 5
   <211> 33
   <212> DNA
   <213> Homo sapiens
<400> 5
   ggcgagatct agcgctatat gcgttgatgc aat 33
<210> 6
   <211> 51
   <212> DNA
   <213> Homo sapiens
<400> 6
   ggccagatct gctaccttaa gagagccgaa acaagcgctc atgagcccga a 51
<210> 7
   <211> 6084
   <212> DNA
   <213> Homo sapiens
<400> 7
   agatcttcaa tattggccat tagccatatt attcattggt tatatagcat aaatcaatat 60
   tggctattgg ccattgcata cgttgtatct atatcataat atgtacattt atattggctc 120
   atgtccaata tgaccgccat gttggcattg attattgact agttattaat agtaatcaat 180
   tacggggtca ttagttcata gcccatatat ggagttccgc gttacataac ttacggtaaa 240
   tggcccgcct ggctgaccgc ccaacgaccc ccgcccattg acgtcaataa tgacgtatgt 300
   tcccatagta acgccaatag ggactttcca ttgacgtcaa tgggtggagt atttacggta 360
   aactgcccac ttggcagtac atcaagtgta tcatatgcca agtccgcccc ctattgacgt 420
   caatgacggt aaatggcccg cctggcatta tgcccagtac atgaccttac gggactttcc 480
   tacttggcag tacatctacg tattagtcat cgctattacc atggtgatgc ggttttggca 540
   gtacaccaat gggcgtggat agcggtttga ctcacgggga tttccaagtc tccaccccat 600
   tgacgtcaat gggagtttgt tttggcacca aaatcaacgg gactttccaa aatgtcgtaa 660
   caactgcgat cgcccgcccc gttgacgcaa atgggcggta ggcgtgtacg gtgggaggtc 720
   tatataagca gagctcgttt agtgaaccgt cagatcacta gaagctttat tgcggtagtt 780
   tatcacagtt aaattgctaa cgcagtcagt gcttctgaca caacagtctc gaacttaagc 840
   tgcagtgact ctcttaatta actccaccag tctcacttca gttccttttg cctccaccag 900
   tctcacttca gttccttttg catgaagagc tcagaatcaa aagaggaaac caacccctaa 960
   gatgagcttt ccatgtaaat ttgtagccag cttccttctg attttcaatg tttcttccaa 1020
   aggtgcagtc tccaaagaga ttacgaatgc cttggaaacc tggggtgcct tgggtcagga 1080
   catcaacttg gacattccta gttttcaaat gagtgatgat attgacgata taaaatggga 1140
   aaaaacttca gacaagaaaa agattgcaca attcagaaaa gagaaagaga ctttcaagga 1200
   aaaagataca tataagctat ttaaaaatgg aactctgaaa attaagcatc tgaagaccga 1260
   tgatcaggat atctacaagg tatcaatata tgatacaaaa ggaaaaaatg tgttggaaaa 1320
   aatatttgat ttgaagattc aagagagggt ctcaaaacca aagatctcct ggacttgtat 1380
   caacacaacc ctgacctgtg aggtaatgaa tggaactgac cccgaattaa acctgtatca 1440
   agatgggaaa catctaaaac tttctcagag ggtcatcaca cacaagtgga ccaccagcct 1500
   gagtgcaaaa ttcaagtgca cagcagggaa caaagtcagc aaggaatcca gtgtcgagcc 1560
   tgtcagctgt ccagagaaag ggatccaggt gagtagggcc cgatccttct agagtcgagc 1620
   tctcttaagg tagcaaggtt acaagacagg tttaaggaga ccaatagaaa ctgggcttgt 1680
   cgagacagag aagactcttg cgtttctgat aggcacctat tggtcttacg cggccgcgaa 1740
   ttccaagctt gagtattcta tcgtgtcacc taaataactt ggcgtaatca tggtcatatc 1800
   tgtttcctgt gtgaaattgt tatccgctca caattccaca caacatacga gccggaagca 1860
   taaagtgtaa agcctggggt gcctaatgag tgagctaact cacattaatt gcgttgcgcg 1920
   atgcttccat tttgtgaggg ttaatgcttc gagaagacat gataagatac attgatgagt 1980
   ttggacaaac cacaacaaga atgcagtgaa aaaaatgctt tatttgtgaa atttgtgatg 2040
   ctattgcttt atttgtaacc attataagct gcaataaaca agttaacaac aacaattgca 2100
   ttcattttat gtttcaggtt cagggggaga tgtgggaggt tttttaaagc aagtaaaacc 2160
   tctacaaatg tggtaaaatc cgataaggat cgattccgga gcctgaatgg cgaatggacg 2220
   cgccctgtag cggcgcatta agcgcggcgg gtgtggtggt tacgcgcacg tgaccgctac 2280
   acttgccagc gccctagcgc ccgctccttt cgctttcttc ccttcctttc tcgccacgtt 2340
   cgccggcttt ccccgtcaag ctctaaatcg ggggctccct ttagggttcc gatttagtgc 2400
   tttacggcac ctcgacccca aaaaacttga ttagggtgat ggttcacgta gtgggccatc 2460
   gccctgatag acggtttttc gccctttgac gttggagtcc acgttcttta atagtggact 2520
   cttgttccaa actggaacaa cactcaaccc tatctcggtc tattcttttg atttataagg 2580
   gattttgccg atttcggcct attggttaaa aaatgagctg atttaacaaa aatttaacgc 2640
   gaattttaac aaaatattaa cgcttacaat ttcgcctgtg taccttctga ggcggaaaga 2700
   accagctgtg gaatgtgtgt cagttagggt gtggaaagtc cccaggctcc ccagcaggca 2760
   gaagtatgca aagcatgcat ctcaattagt cagcaaccag gtgtggaaag tccccaggct 2820
   ccccagcagg cagaagtatg caaagcatgc atctcaatta gtcagcaacc atagtcccgc 2880
   ccctaactcc gcccatcccg cccctaactc cgcccagttc cgcccattct ccgccccatg 2940
   gctgactaat tttttttatt tatgcagagg ccgaggccgc ctcggcctct gagctattcc 3000
   agaagtagtg aggaggcttt tttggaggcc taggcttttg caaaaagctt gattcttctg 3060
   acacaacagt ctcgaactta aggctagagc caccatgatt gaacaagatg gattgcacgc 3120
   aggttctccg gccgcttggg tggagaggct attcggctat gactgggcac aacagacaat 3180
   cggctgctct gatgccgccg tgttccggct gtcagcgcag gggcgcccgg ttctttttgt 3240
   caagaccgac ctgtccggtg ccctgaatga actgcaggac gaggcagcgc ggctatcgtg 3300
   gctggccacg acgggcgttc cttgcgcagc tgtgctcgac gttgtcactg aagcgggaag 3360
   ggactggctg ctattgggcg aagtgccggg gcaggatctc ctgtcatctc accttgctcc 3420
   tgccgagaaa gtatccatca tggctgatgc aatgcggcgg ctgcatacgc ttgatccggc 3480
   tacctgccca ttcgaccacc aagcgaaaca tcgcatcgag cgagcacgta ctcggatgga 3540
   agccggtctt gtcgatcagg atgatctgga cgaagagcat caggggctcg cgccagccga 3600
   actgttcgcc aggctcaagg cgcgcatgcc cgacggcgag gatctcgtcg tgacccatgg 3660
   cgatgcctgc ttgccgaata tcatggtgga aaatggccgc ttttctggat tcatcgactg 3720
   tggccggctg ggtgtggcgg accgctatca ggacatagcg ttggctaccc gtgatattgc 3780
   tgaagagctt ggcggcgaat gggctgaccg cttcctcgtg ctttacggta tcgccgctcc 3840
   cgattcgcag cgcatcgcct tctatcgcct tcttgacgag ttcttctgag cgggactctg 3900
   gggttcgaaa tgaccgacca agcgacgccc aacctgccat cacgatggcc gcaataaaat 3960
   atctttattt tcattacatc tgtgtgttgg ttttttgtgt gaagatccgc gtatggtgca 4020
   ctctcagtac aatctgctct gatgccgcat agttaagcca gccccgacac ccgccaacac 4080
   ccgctgacgc gccctgacgg gcttgtctgc tcccggcatc cgcttacaga caagctgtga 4140
   ccgtctccgg gagctgcatg tgtcagaggt tttcaccgtc atcaccgaaa cgcgcgagac 4200
   gaaagggcct cgtgatacgc ctatttttat aggttaatgt catgataata atggtttctt 4260
   agacgtcagg tggcactttt cggggaaatg tgcgcggaac ccctatttgt ttatttttct 4320
   aaatacattc aaatatgtat ccgctcatga gacaataacc ctgataaatg cttcaataat 4380
   attgaaaaag gaagagtatg agtattcaac atttccgtgt cgcccttatt cccttttttg 4440
   cggcattttg ccttcctgtt tttgctcacc cagaaacgct ggtgaaagta aaagatgctg 4500
   aagatcagtt gggtgcacga gtgggttaca tcgaactgga tctcaacagc ggtaagatcc 4560
   ttgagagttt tcgccccgaa gaacgttttc caatgatgag cacttttaaa gttctgctat 4620
   gtggcgcggt attatcccgt attgacgccg ggcaagagca actcggtcgc cgcatacact 4680
   attctcagaa tgacttggtt gagtactcac cagtcacaga aaagcatctt acggatggca 4740
   tgacagtaag agaattatgc agtgctgcca taaccatgag tgataacact gcggccaact 4800
   tacttctgac aacgatcgga ggaccgaagg agctaaccgc ttttttgcac aacatggggg 4860
   atcatgtaac tcgccttgat cgttgggaac cggagctgaa tgaagccata ccaaacgacg 4920
   agcgtgacac cacgatgcct gtagcaatgg caacaacgtt gcgcaaacta ttaactggcg 4980
   aactacttac tctagcttcc cggcaacaat taatagactg gatggaggcg gataaagttg 5040
   caggaccact tctgcgctcg gcccttccgg ctggctggtt tattgctgat aaatctggag 5100
   ccggtgagcg tgggtctcgc ggtatcattg cagcactggg gccagatggt aagccctccc 5160
   gtatcgtagt tatctacacg acggggagtc aggcaactat ggatgaacga aatagacaga 5220
   tcgctgagat aggtgcctca ctgattaagc attggtaact gtcagaccaa gtttactcat 5280
   atatacttta gattgattta aaacttcatt tttaatttaa aaggatctag gtgaagatcc 5340
   tttttgataa tctcatgacc aaaatccctt aacgtgagtt ttcgttccac tgagcgtcag 5400
   accccgtaga aaagatcaaa ggatcttctt gagatccttt ttttctgcgc gtaatctgct 5460
   gcttgcaaac aaaaaaacca ccgctaccag cggtggtttg tttgccggat caagagctac 5520
   caactctttt tccgaaggta actggcttca gcagagcgca gataccaaat actgtccttc 5580
   tagtgtagcc gtagttaggc caccacttca agaactctgt agcaccgcct acatacctcg 5640
   ctctgctaat cctgttacca gtggctgctg ccagtggcga taagtcgtgt cttaccgggt 5700
   tggactcaag acgatagtta ccggataagg cgcagcggtc gggctgaacg gggggttcgt 5760
   gcacacagcc cagcttggag cgaacgacct acaccgaact gagataccta cagcgtgagc 5820
   tatgagaaag cgccacgctt cccgaaggga gaaaggcgga caggtatccg gtaagcggca 5880
   gggtcggaac aggagagcgc acgagggagc ttccaggggg aaacgcctgg tatctttata 5940
   gtcctgtcgg gtttcgccac ctctgacttg agcgtcgatt tttgtgatgc tcgtcagggg 6000
   ggcggagcct atggaaaaac gccagcaacg cggccttttt acggttcctg gccttttgct 6060
   ggccttttgc tcacatggct cgac 6084
<210> 8
   <211> 6085
   <212> DNA
   <213> Homo sapiens
<400> 8
   agatcttcaa tattggccat tagccatatt attcattggt tatatagcat aaatcaatat 60
   tggctattgg ccattgcata cgttgtatct atatcataat atgtacattt atattggctc 120
   atgtccaata tgaccgccat gttggcattg attattgact agttattaat agtaatcaat 180
   tacggggtca ttagttcata gcccatatat ggagttccgc gttacataac ttacggtaaa 240
   tggcccgcct ggctgaccgc ccaacgaccc ccgcccattg acgtcaataa tgacgtatgt 300
   tcccatagta acgccaatag ggactttcca ttgacgtcaa tgggtggagt atttacggta 360
   aactgcccac ttggcagtac atcaagtgta tcatatgcca agtccgcccc ctattgacgt 420
   caatgacggt aaatggcccg cctggcatta tgcccagtac atgaccttac gggactttcc 480
   tacttggcag tacatctacg tattagtcat cgctattacc atggtgatgc ggttttggca 540
   gtacaccaat gggcgtggat agcggtttga ctcacgggga tttccaagtc tccaccccat 600
   tgacgtcaat gggagtttgt tttggcacca aaatcaacgg gactttccaa aatgtcgtaa 660
   caactgcgat cgcccgcccc gttgacgcaa atgggcggta ggcgtgtacg gtgggaggtc 720
   tatataagca gagctcgttt agtgaaccgt cagatcacta gaagctttat tgcggtagtt 780
   tatcacagtt aaattgctaa cgcagtcagt gcttctgaca caacagtctc gaacttaagc 840
   tgcagtgact ctcttaatta actccaccag tctcacttca gttccttttg cctccaccag 900
   tctcacttca gttccttttg catgaagagc tcagaatcaa aagaggaaac caacccctaa 960
   gatgagcttt ccatgtaaat ttgtagccag cttccttctg attttcaatg tttcttccaa 1020
   aggtgcagtc tccaaagaga ttacgaatgc cttggaaacc tggggtgcct tgggtcagga 1080
   catcaacttg gacattccta gttttcaaat gagtgatgat attgacgata taaaatggga 1140
   aaaaacttca gacaagaaaa agattgcaca attcagaaaa gagaaagaga ctttcaagga 1200
   aaaagataca tataagctat ttaaaaatgg aactctgaaa attaagcatc tgaagaccga 1260
   tgatcaggat atctacaagg tatcaatata tgatacaaaa ggaaaaaatg tgttggaaaa 1320
   aatatttgat ttgaagattc aagagagggt ctcaaaacca aagatctcct ggacttgtat 1380
   caacacaacc ctgacctgtg aggtaatgaa tggaactgac cccgaattaa acctgtatca 1440
   agatgggaaa catctaaaac tttctcagag ggtcatcaca cacaagtgga ccaccagcct 1500
   gagtgcaaaa ttcaagtgca cagcagggaa caaagtcagc aaggaatcca gtgtcgagcc 1560
   tgtcagctgt ccagagaaag ggatcccagg tgagtagggc ccgatccttc tagagtcgag 1620
   ctctcttaag gtagcaaggt tacaagacag gtttaaggag accaatagaa actgggcttg 1680
   tcgagacaga gaagactctt gcgtttctga taggcaccta ttggtcttac gcggccgcga 1740
   attccaagct tgagtattct atcgtgtcac ctaaataact tggcgtaatc atggtcatat 1800
   ctgtttcctg tgtgaaattg ttatccgctc acaattccac acaacatacg agccggaagc 1860
   ataaagtgta aagcctgggg tgcctaatga gtgagctaac tcacattaat tgcgttgcgc 1920
   gatgcttcca ttttgtgagg gttaatgctt cgagaagaca tgataagata cattgatgag 1980
   tttggacaaa ccacaacaag aatgcagtga aaaaaatgct ttatttgtga aatttgtgat 2040
   gctattgctt tatttgtaac cattataagc tgcaataaac aagttaacaa caacaattgc 2100
   attcatttta tgtttcaggt tcagggggag atgtgggagg ttttttaaag caagtaaaac 2160
   ctctacaaat gtggtaaaat ccgataagga tcgattccgg agcctgaatg gcgaatggac 2220
   gcgccctgta gcggcgcatt aagcgcggcg ggtgtggtgg ttacgcgcac gtgaccgcta 2280
   cacttgccag cgccctagcg cccgctcctt tcgctttctt cccttccttt ctcgccacgt 2340
   tcgccggctt tccccgtcaa gctctaaatc gggggctccc tttagggttc cgatttagtg 2400
   ctttacggca cctcgacccc aaaaaacttg attagggtga tggttcacgt agtgggccat 2460
   cgccctgata gacggttttt cgccctttga cgttggagtc cacgttcttt aatagtggac 2520
   tcttgttcca aactggaaca acactcaacc ctatctcggt ctattctttt gatttataag 2580
   ggattttgcc gatttcggcc tattggttaa aaaatgagct gatttaacaa aaatttaacg 2640
   cgaattttaa caaaatatta acgcttacaa tttcgcctgt gtaccttctg aggcggaaag 2700
   aaccagctgt ggaatgtgtg tcagttaggg tgtggaaagt ccccaggctc cccagcaggc 2760
   agaagtatgc aaagcatgca tctcaattag tcagcaacca ggtgtggaaa gtccccaggc 2820
   tccccagcag gcagaagtat gcaaagcatg catctcaatt agtcagcaac catagtcccg 2880
   cccctaactc cgcccatccc gcccctaact ccgcccagtt ccgcccattc tccgccccat 2940
   ggctgactaa ttttttttat ttatgcagag gccgaggccg cctcggcctc tgagctattc 3000
   cagaagtagt gaggaggctt ttttggaggc ctaggctttt gcaaaaagct tgattcttct 3060
   gacacaacag tctcgaactt aaggctagag ccaccatgat tgaacaagat ggattgcacg 3120
   caggttctcc ggccgcttgg gtggagaggc tattcggcta tgactgggca caacagacaa 3180
   tcggctgctc tgatgccgcc gtgttccggc tgtcagcgca ggggcgcccg gttctttttg 3240
   tcaagaccga cctgtccggt gccctgaatg aactgcagga cgaggcagcg cggctatcgt 3300
   ggctggccac gacgggcgtt ccttgcgcag ctgtgctcga cgttgtcact gaagcgggaa 3360
   gggactggct gctattgggc gaagtgccgg ggcaggatct cctgtcatct caccttgctc 3420
   ctgccgagaa agtatccatc atggctgatg caatgcggcg gctgcatacg cttgatccgg 3480
   ctacctgccc attcgaccac caagcgaaac atcgcatcga gcgagcacgt actcggatgg 3540
   aagccggtct tgtcgatcag gatgatctgg acgaagagca tcaggggctc gcgccagccg 3600
   aactgttcgc caggctcaag gcgcgcatgc ccgacggcga ggatctcgtc gtgacccatg 3660
   gcgatgcctg cttgccgaat atcatggtgg aaaatggccg cttttctgga ttcatcgact 3720
   gtggccggct gggtgtggcg gaccgctatc aggacatagc gttggctacc cgtgatattg 3780
   ctgaagagct tggcggcgaa tgggctgacc gcttcctcgt gctttacggt atcgccgctc 3840
   ccgattcgca gcgcatcgcc ttctatcgcc ttcttgacga gttcttctga gcgggactct 3900
   ggggttcgaa atgaccgacc aagcgacgcc caacctgcca tcacgatggc cgcaataaaa 3960
   tatctttatt ttcattacat ctgtgtgttg gttttttgtg tgaagatccg cgtatggtgc 4020
   actctcagta caatctgctc tgatgccgca tagttaagcc agccccgaca cccgccaaca 4080
   cccgctgacg cgccctgacg ggcttgtctg ctcccggcat ccgcttacag acaagctgtg 4140
   accgtctccg ggagctgcat gtgtcagagg ttttcaccgt catcaccgaa acgcgcgaga 4200
   cgaaagggcc tcgtgatacg cctattttta taggttaatg tcatgataat aatggtttct 4260
   tagacgtcag gtggcacttt tcggggaaat gtgcgcggaa cccctatttg tttatttttc 4320
   taaatacatt caaatatgta tccgctcatg agacaataac cctgataaat gcttcaataa 4380
   tattgaaaaa ggaagagtat gagtattcaa catttccgtg tcgcccttat tccctttttt 4440
   gcggcatttt gccttcctgt ttttgctcac ccagaaacgc tggtgaaagt aaaagatgct 4500
   gaagatcagt tgggtgcacg agtgggttac atcgaactgg atctcaacag cggtaagatc 4560
   cttgagagtt ttcgccccga agaacgtttt ccaatgatga gcacttttaa agttctgcta 4620
   tgtggcgcgg tattatcccg tattgacgcc gggcaagagc aactcggtcg ccgcatacac 4680
   tattctcaga atgacttggt tgagtactca ccagtcacag aaaagcatct tacggatggc 4740
   atgacagtaa gagaattatg cagtgctgcc ataaccatga gtgataacac tgcggccaac 4800
   ttacttctga caacgatcgg aggaccgaag gagctaaccg cttttttgca caacatgggg 4860
   gatcatgtaa ctcgccttga tcgttgggaa ccggagctga atgaagccat accaaacgac 4920
   gagcgtgaca ccacgatgcc tgtagcaatg gcaacaacgt tgcgcaaact attaactggc 4980
   gaactactta ctctagcttc ccggcaacaa ttaatagact ggatggaggc ggataaagtt 5040
   gcaggaccac ttctgcgctc ggcccttccg gctggctggt ttattgctga taaatctgga 5100
   gccggtgagc gtgggtctcg cggtatcatt gcagcactgg ggccagatgg taagccctcc 5160
   cgtatcgtag ttatctacac gacggggagt caggcaacta tggatgaacg aaatagacag 5220
   atcgctgaga taggtgcctc actgattaag cattggtaac tgtcagacca agtttactca 5280
   tatatacttt agattgattt aaaacttcat ttttaattta aaaggatcta ggtgaagatc 5340
   ctttttgata atctcatgac caaaatccct taacgtgagt tttcgttcca ctgagcgtca 5400
   gaccccgtag aaaagatcaa aggatcttct tgagatcctt tttttctgcg cgtaatctgc 5460
   tgcttgcaaa caaaaaaacc accgctacca gcggtggttt gtttgccgga tcaagagcta 5520
   ccaactcttt ttccgaaggt aactggcttc agcagagcgc agataccaaa tactgtcctt 5580
   ctagtgtagc cgtagttagg ccaccacttc aagaactctg tagcaccgcc tacatacctc 5640
   gctctgctaa tcctgttacc agtggctgct gccagtggcg ataagtcgtg tcttaccggg 5700
   ttggactcaa gacgatagtt accggataag gcgcagcggt cgggctgaac ggggggttcg 5760
   tgcacacagc ccagcttgga gcgaacgacc tacaccgaac tgagatacct acagcgtgag 5820
   ctatgagaaa gcgccacgct tcccgaaggg agaaaggcgg acaggtatcc ggtaagcggc 5880
   agggtcggaa caggagagcg cacgagggag cttccagggg gaaacgcctg gtatctttat 5940
   agtcctgtcg ggtttcgcca cctctgactt gagcgtcgat ttttgtgatg ctcgtcaggg 6000
   gggcggagcc tatggaaaaa cgccagcaac gcggcctttt tacggttcct ggccttttgc 6060
   tggccttttg ctcacatggc tcgac                                       6085
<210> 9
   <211> 6086
   <212> DNA
   <213> Homo sapiens
<400> 9
   agatcttcaa tattggccat tagccatatt attcattggt tatatagcat aaatcaatat 60
   tggctattgg ccattgcata cgttgtatct atatcataat atgtacattt atattggctc 120
   atgtccaata tgaccgccat gttggcattg attattgact agttattaat agtaatcaat 180
   tacggggtca ttagttcata gcccatatat ggagttccgc gttacataac ttacggtaaa 240
   tggcccgcct ggctgaccgc ccaacgaccc ccgcccattg acgtcaataa tgacgtatgt 300
   tcccatagta acgccaatag ggactttcca ttgacgtcaa tgggtggagt atttacggta 360
   aactgcccac ttggcagtac atcaagtgta tcatatgcca agtccgcccc ctattgacgt 420
   caatgacggt aaatggcccg cctggcatta tgcccagtac atgaccttac gggactttcc 480
   tacttggcag tacatctacg tattagtcat cgctattacc atggtgatgc ggttttggca 540
   gtacaccaat gggcgtggat agcggtttga ctcacgggga tttccaagtc tccaccccat 600
   tgacgtcaat gggagtttgt tttggcacca aaatcaacgg gactttccaa aatgtcgtaa 660
   caactgcgat cgcccgcccc gttgacgcaa atgggcggta ggcgtgtacg gtgggaggtc 720
   tatataagca gagctcgttt agtgaaccgt cagatcacta gaagctttat tgcggtagtt 780
   tatcacagtt aaattgctaa cgcagtcagt gcttctgaca caacagtctc gaacttaagc 840
   tgcagtgact ctcttaatta actccaccag tctcacttca gttccttttg cctccaccag 900
   tctcacttca gttccttttg catgaagagc tcagaatcaa aagaggaaac caacccctaa 960
   gatgagcttt ccatgtaaat ttgtagccag cttccttctg attttcaatg tttcttccaa 1020
   aggtgcagtc tccaaagaga ttacgaatgc cttggaaacc tggggtgcct tgggtcagga 1080
   catcaacttg gacattccta gttttcaaat gagtgatgat attgacgata taaaatggga 1140
   aaaaacttca gacaagaaaa agattgcaca attcagaaaa gagaaagaga ctttcaagga 1200
   aaaagataca tataagctat ttaaaaatgg aactctgaaa attaagcatc tgaagaccga 1260
   tgatcaggat atctacaagg tatcaatata tgatacaaaa ggaaaaaatg tgttggaaaa 1320
   aatatttgat ttgaagattc aagagagggt ctcaaaacca aagatctcct ggacttgtat 1380
   caacacaacc ctgacctgtg aggtaatgaa tggaactgac cccgaattaa acctgtatca 1440
   agatgggaaa catctaaaac tttctcagag ggtcatcaca cacaagtgga ccaccagcct 1500
   gagtgcaaaa ttcaagtgca cagcagggaa caaagtcagc aaggaatcca gtgtcgagcc 1560
   tgtcagctgt ccagagaaag ggatccacag gtgagtaggg cccgatcctt ctagagtcga 1620
   gctctcttaa ggtagcaagg ttacaagaca ggtttaagga gaccaataga aactgggctt 1680
   gtcgagacag agaagactct tgcgtttctg ataggcacct attggtctta cgcggccgcg 1740
   aattccaagc ttgagtattc tatcgtgtca cctaaataac ttggcgtaat catggtcata 1800
   tctgtttcct gtgtgaaatt gttatccgct cacaattcca cacaacatac gagccggaag 1860
   cataaagtgt aaagcctggg gtgcctaatg agtgagctaa ctcacattaa ttgcgttgcg 1920
   cgatgcttcc attttgtgag ggttaatgct tcgagaagac atgataagat acattgatga 1980
   gtttggacaa accacaacaa gaatgcagtg aaaaaaatgc tttatttgtg aaatttgtga 2040
   tgctattgct ttatttgtaa ccattataag ctgcaataaa caagttaaca acaacaattg 2100
   cattcatttt atgtttcagg ttcaggggga gatgtgggag gttttttaaa gcaagtaaaa 2160
   cctctacaaa tgtggtaaaa tccgataagg atcgattccg gagcctgaat ggcgaatgga 2220
   cgcgccctgt agcggcgcat taagcgcggc gggtgtggtg gttacgcgca cgtgaccgct 2280
   acacttgcca gcgccctagc gcccgctcct ttcgctttct tcccttcctt tctcgccacg 2340
   ttcgccggct ttccccgtca agctctaaat cgggggctcc ctttagggtt ccgatttagt 2400
   gctttacggc acctcgaccc caaaaaactt gattagggtg atggttcacg tagtgggcca 2460
   tcgccctgat agacggtttt tcgccctttg acgttggagt ccacgttctt taatagtgga 2520
   ctcttgttcc aaactggaac aacactcaac cctatctcgg tctattcttt tgatttataa 2580
   gggattttgc cgatttcggc ctattggtta aaaaatgagc tgatttaaca aaaatttaac 2640
   gcgaatttta acaaaatatt aacgcttaca atttcgcctg tgtaccttct gaggcggaaa 2700
   gaaccagctg tggaatgtgt gtcagttagg gtgtggaaag tccccaggct ccccagcagg 2760
   cagaagtatg caaagcatgc atctcaatta gtcagcaacc aggtgtggaa agtccccagg 2820
   ctccccagca ggcagaagta tgcaaagcat gcatctcaat tagtcagcaa ccatagtccc 2880
   gcccctaact ccgcccatcc cgcccctaac tccgcccagt tccgcccatt ctccgcccca 2940
   tggctgacta atttttttta tttatgcaga ggccgaggcc gcctcggcct ctgagctatt 3000
   ccagaagtag tgaggaggct tttttggagg cctaggcttt tgcaaaaagc ttgattcttc 3060
   tgacacaaca gtctcgaact taaggctaga gccaccatga ttgaacaaga tggattgcac 3120
   gcaggttctc cggccgcttg ggtggagagg ctattcggct atgactgggc acaacagaca 3180
   atcggctgct ctgatgccgc cgtgttccgg ctgtcagcgc aggggcgccc ggttcttttt 3240
   gtcaagaccg acctgtccgg tgccctgaat gaactgcagg acgaggcagc gcggctatcg 3300
   tggctggcca cgacgggcgt tccttgcgca gctgtgctcg acgttgtcac tgaagcggga 3360
   agggactggc tgctattggg cgaagtgccg gggcaggatc tcctgtcatc tcaccttgct 3420
   cctgccgaga aagtatccat catggctgat gcaatgcggc ggctgcatac gcttgatccg 3480
   gctacctgcc cattcgacca ccaagcgaaa catcgcatcg agcgagcacg tactcggatg 3540
   gaagccggtc ttgtcgatca ggatgatctg gacgaagagc atcaggggct cgcgccagcc 3600
   gaactgttcg ccaggctcaa ggcgcgcatg cccgacggcg aggatctcgt cgtgacccat 3660
   ggcgatgcct gcttgccgaa tatcatggtg gaaaatggcc gcttttctgg attcatcgac 3720
   tgtggccggc tgggtgtggc ggaccgctat caggacatag cgttggctac ccgtgatatt 3780
   gctgaagagc ttggcggcga atgggctgac cgcttcctcg tgctttacgg tatcgccgct 3840
   cccgattcgc agcgcatcgc cttctatcgc cttcttgacg agttcttctg agcgggactc 3900
   tggggttcga aatgaccgac caagcgacgc ccaacctgcc atcacgatgg ccgcaataaa 3960
   atatctttat tttcattaca tctgtgtgtt ggttttttgt gtgaagatcc gcgtatggtg 4020
   cactctcagt acaatctgct ctgatgccgc atagttaagc cagccccgac acccgccaac 4080
   acccgctgac gcgccctgac gggcttgtct gctcccggca tccgcttaca gacaagctgt 4140
   gaccgtctcc gggagctgca tgtgtcagag gttttcaccg tcatcaccga aacgcgcgag 4200
   acgaaagggc ctcgtgatac gcctattttt ataggttaat gtcatgataa taatggtttc 4260
   ttagacgtca ggtggcactt ttcggggaaa tgtgcgcgga acccctattt gtttattttt 4320
   ctaaatacat tcaaatatgt atccgctcat gagacaataa ccctgataaa tgcttcaata 4380
   atattgaaaa aggaagagta tgagtattca acatttccgt gtcgccctta ttcccttttt 4440
   tgcggcattt tgccttcctg tttttgctca cccagaaacg ctggtgaaag taaaagatgc 4500
   tgaagatcag ttgggtgcac gagtgggtta catcgaactg gatctcaaca gcggtaagat 4560
   ccttgagagt tttcgccccg aagaacgttt tccaatgatg agcactttta aagttctgct 4620
   atgtggcgcg gtattatccc gtattgacgc cgggcaagag caactcggtc gccgcataca 4680
   ctattctcag aatgacttgg ttgagtactc accagtcaca gaaaagcatc ttacggatgg 4740
   catgacagta agagaattat gcagtgctgc cataaccatg agtgataaca ctgcggccaa 4800
   cttacttctg acaacgatcg gaggaccgaa ggagctaacc gcttttttgc acaacatggg 4860
   ggatcatgta actcgccttg atcgttggga accggagctg aatgaagcca taccaaacga 4920
   cgagcgtgac accacgatgc ctgtagcaat ggcaacaacg ttgcgcaaac tattaactgg 4980
   cgaactactt actctagctt cccggcaaca attaatagac tggatggagg cggataaagt 5040
   tgcaggacca cttctgcgct cggcccttcc ggctggctgg tttattgctg ataaatctgg 5100
   agccggtgag cgtgggtctc gcggtatcat tgcagcactg gggccagatg gtaagccctc 5160
   ccgtatcgta gttatctaca cgacggggag tcaggcaact atggatgaac gaaatagaca 5220
   gatcgctgag ataggtgcct cactgattaa gcattggtaa ctgtcagacc aagtttactc 5280
   atatatactt tagattgatt taaaacttca tttttaattt aaaaggatct aggtgaagat 5340
   cctttttgat aatctcatga ccaaaatccc ttaacgtgag ttttcgttcc actgagcgtc 5400
   agaccccgta gaaaagatca aaggatcttc ttgagatcct ttttttctgc gcgtaatctg 5460
   ctgcttgcaa acaaaaaaac caccgctacc agcggtggtt tgtttgccgg atcaagagct 5520
   accaactctt tttccgaagg taactggctt cagcagagcg cagataccaa atactgtcct 5580
   tctagtgtag ccgtagttag gccaccactt caagaactct gtagcaccgc ctacatacct 5640
   cgctctgcta atcctgttac cagtggctgc tgccagtggc gataagtcgt gtcttaccgg 5700
   gttggactca agacgatagt taccggataa ggcgcagcgg tcgggctgaa cggggggttc 5760
   gtgcacacag cccagcttgg agcgaacgac ctacaccgaa ctgagatacc tacagcgtga 5820
   gctatgagaa agcgccacgc ttcccgaagg gagaaaggcg gacaggtatc cggtaagcgg 5880
   cagggtcgga acaggagagc gcacgaggga gcttccaggg ggaaacgcct ggtatcttta 5940
   tagtcctgtc gggtttcgcc acctctgact tgagcgtcga tttttgtgat gctcgtcagg 6000
   ggggcggagc ctatggaaaa acgccagcaa cgcggccttt ttacggttcc tggccttttg 6060
   ctggcctttt gctcacatgg ctcgac 6086
<210> 10
   <211> 38
   <212> DNA
   <213> Homo sapiens
<400> 10
   tttttttttt ttcgtcagcg gccgcatcnn nntttatt 38
<210> 11
   <211> 25
   <212> DNA
   <213> Homo sapiens
<400> 11
   cagatcacta gaagctttat tgcgg 25
<210> 12
   <211> 20
   <212> DNA
   <213> Homo sapiens
<400> 12
   ttttcgtcag cggccgcatc 20
<210> 13
   <211> 45
   <212> DNA
   <213> Homo sapiens
<400> 13
   actcataggc catagaggcc tatcacagtt aaattgctaa cgcag 45

## Description

### BACKGROUND OF THE INVENTION

### Field of the Invention

The field of the invention is activating gene expression or causing over-expression of a gene by recombination methods *in situ.* The invention relates to expressing an endogenous gene in a cell at levels higher than those normally found in the cell. Expression of the gene is activated or increased following integration, by non-homologous or illegitimate recombination, of a regulatory sequence that activates expression of the gene. The method allows the identification and expression of genes undiscoverable by current methods since no target sequence is necessary for integration. Thus, gene products associated with human disease and development are obtainable from genes that have not been sequenced and indeed, whose existence is unknown, as well as from well-characterized genes. The methods provide gene products from such genes for therapeutic and diagnostic purposes.

### Related Art

Identification and over-expression of novel genes associated with human disease is an important step towards developing new therapeutic drugs. Current approaches to creating libraries of cells for protein over-expression are based on the production and cloning of cDNA. Thus, in order to identify a new gene using this approach, the gene must be expressed in the cells that were used to make the library. The gene also must be expressed at sufficient levels to be adequately represented in the library. This is problematic because many genes are expressed only in very low quantities, in a rare population of cells, or during short developmental periods.

Furthermore, because of the large size of some mRNAs, it is difficult or impossible to produce full length cDNA molecules capable of expressing the biologically active protein. Lack of full-length cDNA molecules has also been observed for small mRNAs and is thought to be related to sequences in the message that are difficult to produce by reverse transcription or that are unstable during propagation in bacteria. As a result, even the most complete cDNA libraries express only a fraction of the entire set of possible genes.

Finally, many cDNA libraries are produced in bacterial vectors. Use of these vectors to express biologically active mammalian proteins is severely limited since most mammalian proteins do not fold correctly and/or are improperly glycosylated in bacteria.

Therefore, a method for creating a more representative library for protein expression, capable of facilitating faithful expression of biologically active proteins, would be extremely valuable.

Current methods for over-expressing proteins involve cloning the gene of interest and placing it, in a construct, next to a suitable promoter/enhancer, polyadenylation signal, and splice site, and introducing the construct into an appropriate host cell.

An alternative approach involves the use of homologous recombination to activate gene expression by targeting a strong promoter or other regulatory sequence to a previously identified gene.

WO 90/14092 describes *in situ* modification of genes, in mammalian cells, encoding proteins of interest. This application describes single-stranded oligonucleotides for site-directed modification of genes encoding proteins of interest. A marker may also be included. However, the methods are limited to providing an oligonucleotide sequence substantially homologous to a target site. Thus, the method requires knowledge of the site required for activation by site-directed modification and homologous recombination. Novel genes are not discoverable by such methods.

WO 91/06667 describes methods for expressing a mammalian gene insitu. With this method, an amplifiable gene is introduced next to a target gene by homologous recombination. When the cell is then grown in the appropriate medium, both the amplifiable gene and the target gene are amplified and there is enhanced expression of the target gene. As above, methods of introducing the amplifiable gene are limited to homologous recombination, and are not useful for activating novel genes whose sequence (or existence) is unknown.

WO 91/01140 describes the inactivation of endogenous genes by modification of cells by homologous recombination. By these methods, homologous recombination is used to modify and inactivate genes and to produce cells which can serve as donors in gene therapy.

WO 92/20808 describes methods for modifying genomic target sites *in situ.* The modifications are described as being small, for example, changing single bases in DNA. The method relies upon genomic modification using homologous DNA for targeting.

WO 92/19255 describes a method for enhancing the expression of a target gene, achieved by homologous recombination in which a DNA sequence is integrated into the genome or large genomic fragment. This modified sequence can then be transferred to a secondary host for expression. An amplifiable gene can be integrated next to the target gene so that the target region can be amplified for enhanced expression. Homologous recombination is necessary to this targeted approach.

WO 93/09222 describes methods of making proteins by activating an endogenous gene encoding a desired product. A regulatory region is targeted by homologous recombination and replacing or disabling the region normally associated with the gene whose expression is desired. This disabling or replacement causes the gene to be expressed at levels higher than normal.

WO 94/12650 describes a method for activating expression of and amplifying an endogenous gene *in situ* in a cell, which gene is not expressed or is not expressed at desired levels in the cell. The cell is transfected with exogenous DNA sequences which repair, alter, delete, or replace a sequence present in the cell or which are regulatory sequences not normally functionally linked to the endogenous gene in the cell. In order to do this, DNA sequences homologous to genomic DNA sequences at a preselected site are used to target the endogenous gene. In addition, amplifiable DNA encoding a selectable marker can be included. By culturing the homologously recombinant cells under conditions that select for amplification, both the endogenous gene and the amplifiable marker are co-amplified and expression of the gene increased.

WO 95/31560 describes DNA constructs for homologous recombination. The constructs include a targeting sequence, a regulatory sequence, an exon, and an unpaired splice donor site. The targeting is achieved by homologous recombination of the construct with genomic sequences in the cell and allows the production of a protein *in vitro* or *in vivo.*

WO 96/29411 describes methods using an exogenous regulatory sequence, an exogenous exon, either coding or non-coding, and a splice donor site introduced into a preselected site in the genome by homologous recombination. In this application, the introduced DNA is positioned so that the transcripts under control of the exogenous regulatory region include both the exogenous exon and endogenous exons present in either the thrombopoietin, DNase I, or β-interferon genes, resulting in transcripts in which the exogenous and exogenous exons are operably linked. The novel transcription units are produced by homologous recombination.

U.S. Patent No. 5,272,071 describes the transcriptional activation of transcriptionally silent genes in a cell by inserting a DNA regulatory element capable of promoting the expression of a gene normally expressed in that cell. The regulatory element is inserted so that it is operably linked to the normally silent gene. The insertion is accomplished by means of homologous recombination by creating a DNA construct with a segment of the normally silent gene (the target DNA) and the DNA regulatory element used to induce the desired transcription.

U.S. Patent No. 5,578,461 discusses activating expression of mammalian target genes by homologous recombination. A DNA sequence is integrated into the genome or a large genomic fragment to enhance the expression of the target gene. The modified construct can then be transferred to a secondary host. An amplifiable gene can be integrated adjacent to the target gene so that the target region is amplified for enhanced expression.

Both of the above approaches (construction of an over-expressing construct by cloning or by homologous recombination *in vivo*) require the gene to be cloned and sequenced before it can be over-expressed. Furthermore, using homologous recombination, the genomic sequence and structure must also be known.

Unfortunately, many genes have not yet been identified and/or sequenced. Thus, a method for over-expressing a gene or interest, whether or not it has been previously cloned, and whether or not its sequence and structure are known, would be useful.

### BRIEF SUMMARY OF THE INVENTION

The invention is, therefore, generally directed to methods for over-expressing an endogenous gene in a cell, comprising introducing a vector containing a transcriptional regulatory sequence into the cell, allowing the vector to integrate into the genome of the cell by non-homologous recombination, and allowing over-expression of the endogenous gene in the cell. The method does not require previous knowledge of the sequence of the endogenous gene or even of the existence of the gene.

Thus, the present invention provides a method for producing a protein product of an endogenous gene comprising culturing a eukaryotic cell under conditions favoring the production of a protein product of an endogenous gene in said eukaryotic cell, said cell made by:
(a) introducing a vector construct comprising a transcriptional regulatory sequence for over-expression operably linked to an unpaired splice donor sequence into a eukaryotic cell *in vitro;*
(b) integrating the vector construct into the genome of said eukaryotic cell by non-homologous recombination; and
(c) over-expressing protein encoded by an endogenous gene in said eukaryotic cell by upregulation of said gene by said transcriptional regulatory sequence.

Also referred to is a novel vector constructs for activating gene expression or over-expressing a gene through non-homologous recombination. The novel construct lacks homologous targeting sequences. That is, it does not contain nucleotide sequences that target host cell DNA and promote homologous recombination at the target site, causing over-expressing of a cellular gene via the introduced transcriptional regulatory sequence.

Novel vector constructs include a vector containing a transcriptional regulatory sequence operably linked to an unpaired splice donor sequence and further contains one or more amplifiable markers.

Novel vector constructs include constructs with a transcriptional regulatory sequence operably linked to a translational start codon, a signal secretion sequence, and an unpaired splice donor site; constructs with a transcriptional regulatory sequence, operably linked to a translation start codon, an epitope tag, and an unpaired splice donor site; constructs containing a transcriptional regulatory sequence operably linked to a translational start codon, a signal sequence and an epitope tag, and an unpaired splice donor site; constructs containing a transcriptional regulatory sequence operably linked to a translation start codon, a signal secretion sequence, an epitope tag, and a sequence-specific protease site, and an unpaired splice donor site.

The vector construct can contain one or more selectable markers for recombinant host cell selection. Alternatively, selection can be effected by phenotypic selection for a trait provided by the activated endogenous gene product.

These vectors, and indeed any ofthe vectors disclosed herein, and variants of the vectors that will be readily recognized by one of ordinary skill in the art, can be used in any of the methods described herein to form any of the compositions producible by these methods.

The transcriptional regulatory sequence used in the vector constructs includes, but is not limited to, a promoter. In preferred embodiments, the promoter is a viral promoter. In highly preferred embodiments, the viral promoter is the cytomegalovirus immediate early promoter. In alternative embodiments, the promoter is a cellular, non-viral promoter or inducible promoter.

The transcriptional regulatory sequence used in the vector construct may also include, but is not limited to, an enhancer. In preferred embodiments, the enhancer is a viral enhancer. In highly preferred embodiments, the viral enhancer is the cytomegalovirus immediate early enhancer. In alternative embodiments, the enhancer is a cellular non-viral enhancer.

In preferred embodiments of the methods described herein, the vector construct be, or may contain, linear RNA or DNA.

The cell containing the vector may be screened for expression of the gene. The cell over-expressing the gene can be cultured *in vitro* under conditions favoring the production, by the cell, of desired amounts of the gene product (also referred to interchangeably herein as the "expression product") of the endogenous gene that has been activated or whose expression has been increased. The expression product can then be isolated and purified to use, for example, in protein therapy or drug discovery.

Alternatively, the cell expressing the desired gene product can be allowed to express the gene product *in vivo.* In certain such aspects, the cell containing a vector construct of the invention integrated into its genome may be introduced into a eukaryote (such as a vertebrate, particularly a mammal, more particularly a human) under conditions favoring the overexpression or activation of the gene by the cell *in vivo* in the eukaryote. In related such aspects , the cell may be isolated and cloned prior to being introduced into the eukaryote.

Also referred to are methods for over-expressing an endogenous gene in a cell, comprising introducing a vector containing a transcriptional regulatory sequence and one or more amplifiable markers into the cell, allowing the vector to integrate into the genome of the cell by non-homologous recombination, and allowing over-expression of the endogenous gene in the cell.

The cell containing the vector may be screened for over-expression of the gene.

The cell over-expressing the gene is cultured such that amplification of the endogenous gene is obtained. The cell can then be cultured *in vitro* so as to produce desired amounts of the gene product of the amplified endogenous gene that has been activated or whose expression has been increased. The gene product can then be isolated and purified.

Alternatively, following amplification, the cell can be allowed to express the endogenous gene and produce desired amounts of the gene product *in vivo.*

It is to be understood, however, that any vector used in the methods described herein can include one or more amplifiable markers. Thereby, amplification of both the vector and the DNA of interest (i.e., containing the over-expressed gene) occurs in the cell, and further enhanced expression of the endogenous gene is obtained. Accordingly, methods can include a step in which the endogenous gene is amplified.

Also referred to are methods for over-expressing an endogenous gene in a cell comprising introducing a vector containing a transcriptional regulatory sequence and an unpaired splice donor sequence into the cell, allowing the vector to integrate into the genome of the cell by non-homologous recombination, and allowing over-expression of the endogenous gene in the cell.

The cell containing the vector may be screened for expression of the gene.

The cell over-expressing the gene can be cultured *in vitro* so as to produce desirable amounts of the gene product of the endogenous gene whose expression has been activated or increased. The gene product can then be isolated and purified.

Alternatively, the cell can be allowed to express the desired gene product *in vitro.*

The vector construct can consist essentially of the transcriptional regulatory sequence.

The vector construct can consist essentially of the transcriptional regulatory sequence and one or more amplifiable markers.

The vector construct can consist essentially of the transcriptional regulatory sequence and the splice donor sequence.

Any of the vector constructs can also include a secretion signal sequence. The secretion signal sequence is arranged in the construct so that it will be operably linked to the activated endogenous protein. Thereby, secretion of the protein of interest occurs in the cell, and purification of that protein is facilitated. Accordingly, methods can include a step in which the protein expression product is secreted from the cell.

Also referred to are cells made by any of the above methods. Also referred to are cells containing the vector constructs, cells in which the vector constructs have integrated into the cellular genome, and cells which are over-expressing desired gene products from an endogenous gene, over-expression being driven by the introduced transcriptional regulatory sequence.

The cells can be isolated and cloned.

The methods can be carried out in any cell of eukaryotic origin, such as fungal, plant or animal. In preferred embodiments, the methods of the invention may be carried out in vertebrate cells, and particularly mammalian cells including but not limited to rat, mouse, bovine, porcine, sheep, goat and human cells, and more particularly in human cells.

A single cell made by the methods described above can over-express a single gene or more than one gene. More than one gene in a cell can be activated by the integration of a single type of construct into multiple locations in the genome. Similarly, more than one gene in a cell can be activated by the integration of multiple constructs (i.e., more than one type of construct) into multiple locations in the genome. Therefore, a cell can contain only one type of vector construct or different types of constructs, each capable of activating an endogenous gene.

The invention is also directed to methods for making the cells described above by one or more of the following: introducing one or more of the vector constructs of the invention into a cell; allowing the introduced construct(s) to integrate into the genome ofthe cell by non-homologous recombination; allowing over-expression of one or more-endogenous genes in the cell; and isolating and cloning the cell. Also referred to are cells produced by such methods, which may be isolated cells.

Also referred to are methods for using the cells described above to over-express a gene, such as an endogenous cellular gene, that has been characterized (for example, sequenced), uncharacterized (for example, a gene whose function is known but which has not been cloned or sequenced), or a gene whose existence was, prior to over-expression, unknown. The cells can be used to produce desired amounts of an expression product *in vitro* or *in vivo.* If desired, this expression product can then be isolated and purified, for example by cell lysis or by isolation from the growth medium (as when the vector contains a secretion signal sequence).

Also referred to are libraries of cells made by the above described methods. A library can encompass all of the clones from a single transfection experiment or a subset of clones from a single transfection experiment. The subset can over-express the same gene or more than one gene, for example, a class of genes. The transfection can have been done with a single construct or with more than one construct.

A library can also be formed by combining all of the recombinant cells from two or more transfection experiments, by combining one or more subsets of cells from a single transfection experiment or by combining subsets of cells from separate transfection experiments. The resulting library can express the same gene, or more than one gene, for example, a class of genes. Again, in each of these individual transfections, a unique construct or more than one construct can be used.

Libraries can be formed from the same cell type or different cell types.

Also referred to are methods for making libraries by selecting various subsets of cells from the same or different transfection experiments.

Also referred to are methods of using the above-described cells or libraries of cells to over-express or activate endogenous genes, or to obtain the gene expression products of such over-expressed or activated genes. According to this aspect, the cell or library may be screened for the expression of the gene and cells that express the desired gene product may be selected. The cell can then be used to isolate or purify the gene product for subsequent use. Expression in the cell can occur by culturing the cell *in vitro,* under conditions favoring the production of the expression product of the endogenous gene by the cell, or by allowing the cell to express the gene *in vivo.*

In preferred embodiments of the invention, the methods include a process wherein the expression product is isolated or purified. In highly preferred embodiments, the cells expressing the endogenous gene product are cultured under conditions favoring production of sufficient amounts of gene product for commercial application, and especially for diagnostic, therapeutic and drug discovery uses.

Any of the methods can further comprise introducing double-strand breaks into the genomic DNA in the cell prior to or simultaneously with vector integration.

Other preferred embodiments of the present invention will be apparent to one of ordinary skill in light of the following drawings and description of the invention, and of the claims.

### BRIEF DESCRIPTION OF THE DRAWINGS

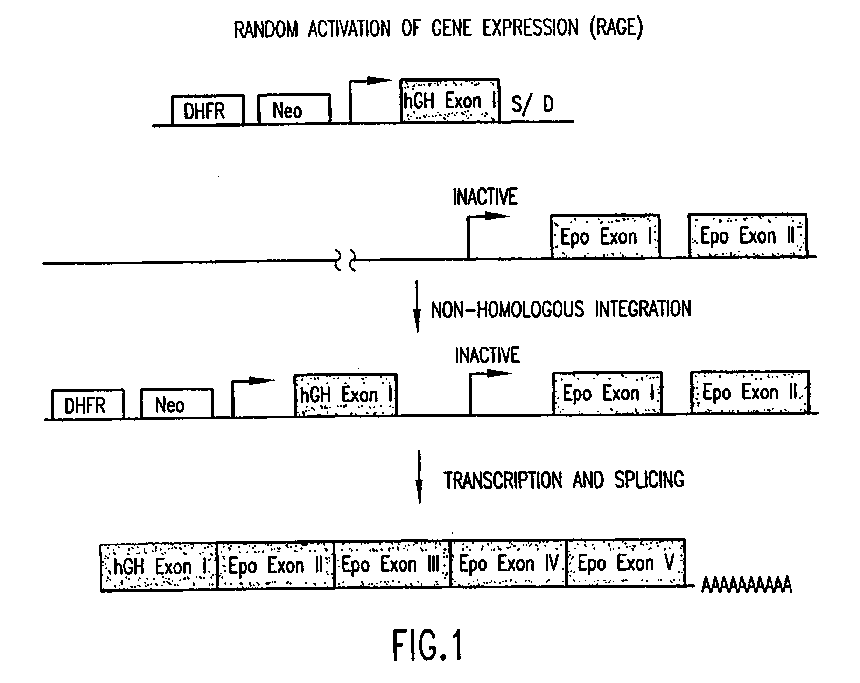

**FIG. 1**. Schematic diagram of gene activation events described herein. The activation construct is transfected into cells and allowed to integrate into the host cell chromosomes at DNA breaks. If breakage occurs upstream of a gene of interest (e.g., Epo), and the appropriate activation construct integrates at the break such that its regulatory sequence becomes operably linked to the gene of interest, activation of the gene will occur. Transcription and splicing produce a chimeric RNA molecule containing exonic sequences from the activation construct and from the endogenous gene. Subsequent translation will result in the production of the protein of interest. Following isolation of the recombinant cell, gene expression can be further enhanced via gene amplification.
**FIG. 2**. Schematic diagram of non-translated activation constructs. The arrows denote promoter sequences. The exonic sequences are shown as open boxes and the splice donor sequence is indicated by S/D. Construct numbers corresponding to the description below are shown on the left. The selectable and amplifiable markers are not shown.
**FIG. 3**. Schematic diagram of translated activation constructs. The arrows denote promoter sequences. The exonic sequences are shown as open boxes and the splice donor sequence is indicated by S/D. The translated, signal peptide, epitope tag, and protease cleavage sequences are shown in the legend below the constructs. Construct numbers corresponding to the description below are shown on the left. The selectable and amplifiable markers are not shown.
**FIG. 4**. Schematic diagram of an activation construct capable of activating endogenous genes.
**FIG. 5A-5D**. Nucleotide sequence of pRIG8R1-CD2 (SEQ ID NO:7).
**FIG. 6A-6C**. Nucleotide sequence of pRIG8R2-CD2 (SEQ ID NO: 8).
**FIG. 7A-7C**. Nucleotide sequence of pRIG8R3-CD2 (SEQ ID NO:9).

### DETAILED DESCRIPTION OF THE INVENTION

There are great advantages of gene activation by non-homologous recombination over other gene activation procedures. Unlike previous methods of protein over-expression, the methods described herein do not require that the gene of interest be cloned (isolated from the cell). Nor do they require any knowledge of the DNA sequence or structure of the gene to be over-expressed (i.e., the sequence of the ORF, introns, exons, or upstream and downstream regulatory elements) or knowledge of a gene's expression patterns (i.e., tissue specificity, developmental regulation, etc.). Furthermore, the methods do not require any knowledge pertaining to the genomic organization of the gene of interest (i.e., the intron and exon structure).

The methods of the present invention thus involve vector constructs that do not contain target nucleotide sequences for homologous recombination. A target sequence allows homologous recombination of vector DNA with cellular DNA at a predetermined site on the cellular DNA, the site having homology for sequences in the vector, the homologous recombination at the predetermined site resulting in the introduction of the transcriptional regulatory sequence into the genome and the subsequent endogenous gene activation.

The method of the present invention does not involve integration of the vector at predetermined sites. Instead, the present methods involve integration of the vector constructs ofthe invention into cellular DNA (*e.g*., the cellular genome) by nonhomologous or "illegitimate" recombination.

The vectors described herein do not contain target sequences. A target sequence is a sequence on the vector that has homology with a sequence or sequences within the gene to be activated or upstream of the gene to be activated, the upstream region being up to and including the first functional splice acceptor site on the same coding strand of the gene of interest, and by means of which homology the transcriptional regulatory sequence that activates the gene of interest is integrated into the genome of the cell containing the gene to be activated. In the case of an enhancer integration vector for activating an endogenous gene, the vector does not contain homology to any sequence in the genome upstream or downstream of the gene of interest (or within the gene of interest) for a distance extending as far as enhancer function is operative.

The present methods, therefore, are capable of identifying new genes that have been or can be missed using conventional and currently available cloning techniques. By using the constructs and methodology described herein, unknown and/or uncharacterized genes can be rapidly identified and over-expressed to produce proteins. The proteins have use as, among other things, human therapeutics and diagnostics and as targets for drug discovery.

The methods are also capable of producing over-expression of known and/or characterized genes for *in vitro* or *in vivo* protein production.

A "known" gene relates to the level of characterization of a gene. The invention allows expression of genes that have been characterized, as well as expression of genes that have not been characterized. Different levels of characterization are possible. These include detailed characterization, such as cloning, DNA, RNA, and/or protein sequencing, and relating the regulation and function of the gene to the cloned sequence (e.g., recognition of promoter and enhancer sequences, functions of the open reading frames, introns, and the like). Characterization can be less detailed, such as having mapped a gene and related function, or having a partial amino acid or nucleotide sequence, or having purified a protein and ascertained a function. Characterization may be minimal, as when a nucleotide or amino acid sequence is known or a protein has been isolated but the function is unknown. Alternatively, a function may be known but the associated protein or nucleotide sequence is not known or is known but has not been correlated to the function. Finally, there may be no characterization in that both the existence of the gene and its function are not known. The invention allows expression of any gene at any of these or other specific degrees of characterization.

Many different proteins (also referred to herein interchangeably as "gene products" or "expression products") can be activated or over-expressed by a single activation construct and in a single set of transfections. Thus, a single cell or different cells in a set of transfectants (library) can over-express more than one protein following transfection with the same or different constructs. Previous activation methods require a unique construct to be created for each gene to be activated.

Further, many different integration sites adjacent to a single gene can be created and tested simultaneously using a single construct. This allows rapid determination of the optimal genomic location of the activation construct for protein expression.

Using previous methods, the 5' end of the gene of interest had to be extensively characterized with respect to sequence and structure. For each activation construct to be produced, an appropriate targeting sequence had to be isolated. Usually, this must be an isogenic sequence isolated from the same person or laboratory strain of animal as the cells to be activated. In some cases, this DNA may be 50 kb or more from the gene of interest. Thus, production of each targeting construct required an arduous amount of cloning and sequencing of the endogenous gene. However, since sequence and structure information is not required for the methods of the present invention, unknown genes and genes with uncharacterized upstream regions can be activated.

This is made possible using *in situ* gene activation using non- homologous recombination of exogenous DNA sequences with cellular DNA. Methods and compositions (*e.g*., vector constructs) required to accomplish such *in situ* gene activation using non- homologous recombination are provided by the present invention.

DNA molecules can recombine to redistribute their genetic content by several different and distinct mechanisms, including homologous recombination, site-specific recombination, and non-homologous/illegitimate recombination. Homologous recombination involves recombination between stretches of DNA that are highly similar in sequence. It has been demonstrated that homologous recombination involves pairing between the homologous sequences along their length prior to redistribution of the genetic material. The exact site of crossover can be at any point in the homologous segments. The efficiency of recombination is proportional to the length of homologous targeting sequence (Hope, Development 113:399 (1991); Reddy et al., J. Virol. 65:1507 (1991)), the degree of sequence identity between the two recombining sequences (von Melchner et al., GenesDev. 6:919 (1992)), and the ratio of homologous to non-homologous DNA present on the construct (Letson, Genetics 117:759 (1987)).

Site-specific recombination, on the other hand, involves the exchange of genetic material at a predetermined site, designated by specific DNA sequences. In this reaction, a protein recombinase binds to the recombination signal sequences, creates a strand scission, and facilitates DNA strand exchange. *Cre*/*Lox* recombination is an example of site specific recombination.

Non-homologous/illegitimate recombination, such as that used advantageously by the methods of the present invention, involves the joining (exchange or redistribution) of genetic material that does not share significant sequence homology and does not occur at site-specific recombination sequences. Examples of non-homologous recombination include integration of exogenous DNA into chromosomes at non-homologous sites, chromosomal translocations and deletions, DNA end-joining, double strand break repair of chromosome ends, bridge-breakage fusion, and concatemerization oftransfected sequences. In most cases, non-homologous recombination is thought to occur through the joining of "free DNA ends." Free ends are DNA molecules that contain an end capable of being joined to a second DNA end either directly, or following repair or processing. The DNA end may consist of a 5' overhang, 3' overhang, or blunt end.

As used herein, retroviral insertion and other transposition reactions are loosely considered forms of non-homologous recombination. These reactions do not involve the use of homology between the recombining molecules. Furthermore, unlike site-specific recombination, these types of recombination reactions do not occur between discrete sites. Instead, a specific protein/DNA complex is required on only one of the recombination partners (i.e., the retrovirus or transposon), with the second DNA partner (i.e., the cellular genome) usually being relatively non-specific. As a result, these "vectors" do not integrate into the cellular genome in a targeted fashion, and therefore they can be used to deliver the activation construct according to the present invention.

Vector constructs useful for the methods described herein ideally may contain a transcriptional regulatory sequence that undergoes non-homologous recombination with genomic sequences in a cell to over-express an endogenous gene in that cell. The vector constructs also lack homologous targeting sequences. That is, they do not contain DNA sequences that target host cell DNA and promote homologous recombination at the target site. Thus, integration of the vector constructs into the cellular genome occurs by non-homologous recombination, and can lead to over-expression of a cellular gene via the introduced transcriptional regulatory sequence contained on the integrated vector construct.

Also referred to are methods for over-expressing an endogenous gene in a cell, comprising introducing a vector containing a transcriptional regulatory sequence into the cell, allowing the vector to integrate into the genome of the cell by non-homologous recombination, and allowing over-expression ofthe endogenous gene in the cell. The method does not require previous knowledge of the sequence of the endogenous gene or even of the existence of the gene. Where the sequence of the gene to be activated is known, however, the constructs can be engineered to contain the proper configuration of vector elements (e.g., location ofthe start codon, addition of codons present in the first exon of the endogenous gene, and the proper reading frame) to achieve maximal overexpression and/or the appropriate protein sequence.

The cell containing the vector may be screened for expression of the gene.

The cell over-expressing the gene can be cultured *in vitro* under conditions favoring the production, by the cell, of desired amounts of the gene product of the endogenous gene that has been activated or whose expression has been increased. If desired, the gene product can then be isolated or purified to use, for example, in protein therapy or drug discovery.

Alternatively, the cell expressing the desired gene product can be allowed to express the gene product *in vivo*.

The vector construct can consist essentially of the transcriptional regulatory sequence.

Alternatively, the vector construct can consist essentially of the transcriptional regulatory sequence and one or more amplifiable markers.

Also referred to are methods for over-expressing an endogenous gene in a cell, comprising introducing a vector containing a transcriptional regulatory sequence and an amplifiable marker into the cell, allowing the vector to integrate into the genome of the cell by non-homologous recombination, and allowing over-expression of the endogenous gene in the cell.

The cell containing the vector is screened for over-expression of the gene.

The cell over-expressing the gene is cultured such that amplification of the endogenous gene is obtained. The cell can then be cultured *in vitro* so as to produce desired amounts of the gene product of the amplified endogenous gene that has been activated or whose expression has been increased. The gene product can then be isolated and purified.

Alternatively, following amplification, the cell can be allowed to express the endogenous gene and produce desired amounts of the gene product *in vivo.*

The vector construct can consist essentially of the transcriptional regulatory sequence and the splice donor sequence.

Also referred to are methods for over-expressing an endogenous gene in a cell comprising introducing a vector containing a transcriptional regulatory sequence and an unpaired splice donor sequence into the cell, allowing the vector to integrate into the genome of the cell by non-homologous recombination, and allowing over-expression of the endogenous gene in the cell.

The cell containing the vector is screened for expression of the gene.

The cell over-expressing the gene can be cultured *in vitro* so as to produce desirable amounts of the gene product of the endogenous gene whose expression has been activated or increased. The gene product can then be isolated and purified.

Alternatively, the cell can be allowed to express the desired gene product *in vivo.*

The vector construct can consist essentially of a transcriptional regulatory sequence operably linked to an unpaired splice donor sequence and also containing an amplifiable marker.

Other activation vectors include constructs with a transcriptional regulatory sequence and an exonic sequence containing a start codon; a transcriptional regulatory sequence and an exonic sequence containing a translational start codon and a secretion signal sequence; constructs with a transcriptional regulatory sequence and an exonic sequence containing a translation start codon, and an epitope tag; constructs containing a transcriptional regulatory sequence and an exonic sequence containing a translational start codon, a signal sequence and an epitope tag; constructs containing a transcriptional regulatory sequence and an exonic sequence with a translation start codon, a signal secretion sequence, an epitope tag, and a sequence-specific protease site. In each of the above constructs, the exon on the construct is located immediately upstream of an unpaired splice donor site.

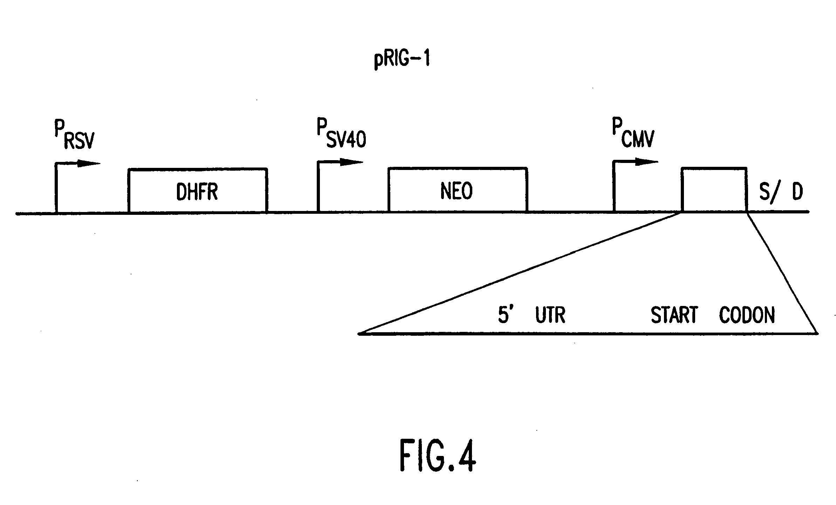

The constructs can also contain a regulatory sequence, a selectable marker lacking a poly A signal, an internal ribosome entry site (ires), and an unpaired splice donor site (FIG. 4). A start codon, signal secretion sequence, epitope tag, and/or a protease cleavage site may optionally be included between the ires and the unpaired splice donor sequence. When this construct integrates upstream of a gene, the selectable marker will be efficiently expressed since a poly A site will be supplied by the endogenous gene. In addition the downstream gene will also be expressed since the ires will allow protein translation to initiate at the downstream open reading frame (i.e. the endogenous gene). Thus, the message produced by this activation construct will be polycistronic. The advantage of this construct is that integration events that do not occur near genes and in the proper orientation will not produce a drug resistant colony. The reason for this is that without a poly A tail (supplied by the endogenous gene), the neomycin resistance gene will not express efficiently. By reducing the number of nonproductive integration events, the complexity of the library can be reduced without affecting its coverage (the number of genes activated), and this will facilitate the screening process.

In another embodiment ofthis construct, *cre-lox* recombination sequences can be included between the regulatory sequence and the *neo* start codon and between the ires and the unpaired splice donor site (between the ires and the start codon, if present). Following isolation of cells that have activated the gene of interest, the *neo* gene and ires can be removed by transfecting the cells with a plasmid encoding the *cre* recombinase. This would eliminate the production ofthe polycistronic message and allow the endogenous gene to be expressed directly from the regulatory sequence on the integrated activation construct. Use of *Cre* recombination to facilitate deletion of genetic elements from mammalian chromosomes has been described (Gu et al., Science 265:103 (1994); Sauer, Meth. Enzymology 225:890-900 (1993)).

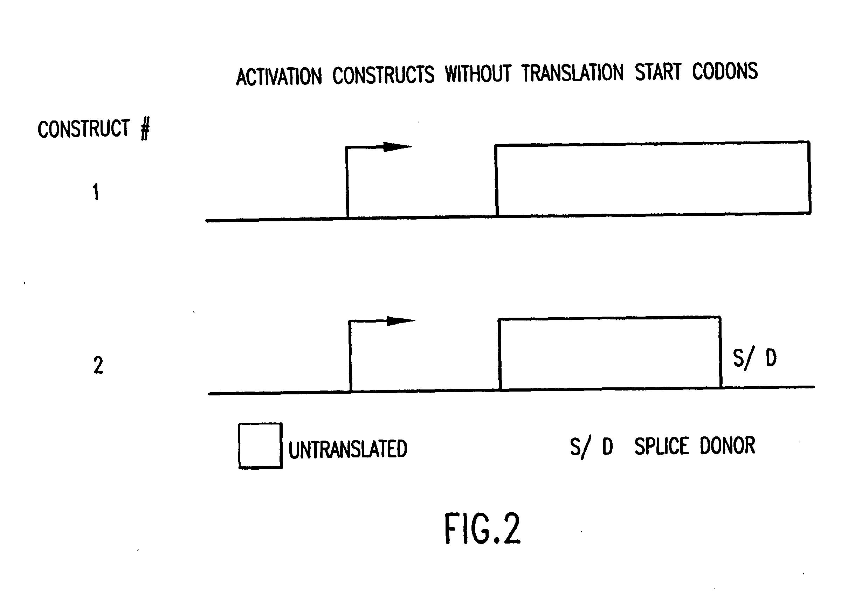

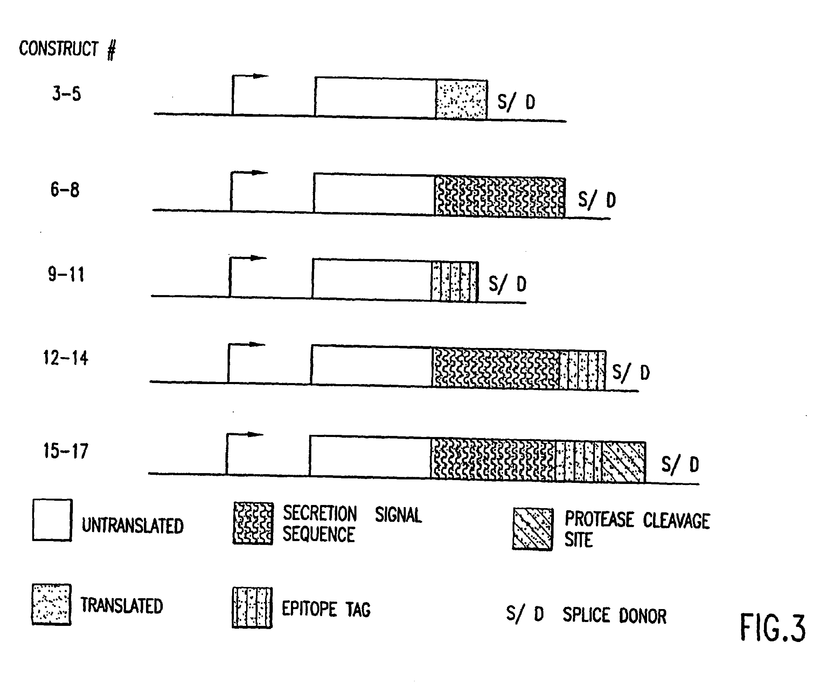

Thus, constructs useful in the methods described herein include, but are not limited to, the following (See also Figures 1-4):
1) Construct with a regulatory sequence and an exon lacking a translation start codon.
2) Construct with a regulatory sequence and an exon lacking a translation start codon followed by a splice donor site.
3) Construct with a regulatory sequence and an exon containing a translation start codon in reading frame 1 (relative to the splice donor site), followed by an unpaired splice donor site.
4) Construct with a regulatory sequence and an exon containing a translation start codon in reading frame 2 (relative to the splice donor site), followed by an unpaired splice donor site.
5) Construct with a regulatory sequence and an exon containing a translation start codon in reading frame 3 (relative to the splice donor site), followed by an unpaired splice donor site.
6) Construct with a regulatory sequence and an exon containing a translation start codon and a signal secretion sequence in reading frame 1 (relative to the splice donor site), followed by an unpaired splice donor site.
7) Construct with a regulatory sequence and an exon containing a translation start codon and a signal secretion sequence in reading frame 2 (relative to the splice donor site), followed by an unpaired splice donor site.
8) Construct with a regulatory sequence and an exon containing a translation start codon and a signal secretion sequence in reading frame 3 (relative to the splice donor site), followed by an unpaired splice donor site.
9) Construct with a regulatory sequence and an exon containing (from 5' to 3') a translation start codon and an epitope tag in reading frame 1 (relative to the splice donor site), followed by an unpaired splice donor site.
10) Construct with a regulatory sequence and an exon containing (from 5' to 3') a translation start codon and an epitope tag in reading frame 2 (relative to the splice donor site), followed by an unpaired splice donor site.
11) Construct with a regulatory sequence and an exon containing (from 5' to 3') a translation start codon and an epitope tag in reading frame 3 (relative to the splice donor site), followed by an unpaired splice donor site.
12) Construct with a regulatory sequence and an exon containing (from 5' to 3') a translation start codon, a signal secretion sequence, and an epitope tag in reading frame 1 (relative to the splice donor site), followed by an unpaired splice donor site.
13) Construct with a regulatory sequence and an exon containing (from 5' to 3') a translation start codon, a signal secretion sequence, and an epitope tag in reading frame 2 (relative to the splice donor site), followed by an unpaired splice donor site.
14) Construct with a regulatory sequence and an exon containing (from 5' to 3') a translation start codon, a signal secretion sequence, and an epitope tag in reading frame 3 (relative to the splice donor site), followed by an unpaired splice donor site.
15) Construct with a regulatory sequence and an exon containing (from 5' to 3') a translation start codon, a signal secretion sequence, an epitope tag, and a sequence specific protease site in reading frame 1 (relative to the splice donor site), followed by an unpaired splice donor site.
16) Construct with a regulatory sequence and an exon containing (from 5' to 3') a translation start codon, a signal secretion sequence, an epitope tag, and a sequence specific protease site in reading frame 2 (relative to the splice donor site), followed by an unpaired splice donor site.
17) Construct with a regulatory sequence and an exon containing (from 5' to 3') a translation start codon, a signal secretion sequence, an epitope tag, and a sequence specific protease site in reading frame 3 (relative to the splice donor site), followed by an unpaired splice donor site.
18) Construct with a regulatory sequence linked to a selectable marker, followed by an internal ribosome entry site, and an unpaired splice donor site.
19) Construct 18 in which a cre/lox recombination signal is located between a) the regulatory sequence and the open reading frame of the selectable marker and b) between the ires and the unpaired splice donor site.
20) Construct with a regulatory sequence operably linked to an exon containing green fluorescent protein lacking a stop codon, followed by an unpaired splice donor site.

It is to be understood, however, that any vector used in the methods described herein can include one or more (i. e., one, two, three, four, five, or more, and most preferably one or two) amplifiable markers. Accordingly, methods can include a step in which the endogenous gene is amplified. Placement of one or more amplifiable markers on the activation construct results in the juxtaposition of the gene of interest and the one or more amplifiable markers in the activated cell. Once the activated cell has been isolated, expression can be further increased by selecting for cells containing an increased copy number of the locus containing both the gene of interest and the activation construct. This can be accomplished by selection methods known in the art, for example by culturing cells in selective culture media containing one or more selection agents that are specific for the one or more amplifiable markers contained on the genetic construct or vector.

Following activation of an endogenous gene by nonhomologous integration of any of the vectors described above, the expression of the endogenous gene may be further increased by selecting for increased copies of the amplifiable marker(s) located on the integrated vector. While such an approach may be accomplished using one amplifiable marker on the integrated vector, in an alternative embodiment the invention provides such methods wherein two or more (*i.e*., two, three, four, five, or more, and most preferably two) amplifiable markers may be included on the vector to facilitate more efficient selection of cells that have amplified the vector and flanking gene of interest. This approach is particularly useful in cells that have a functional endogenous copy of one or more of the amplifiable marker(s) that are contained on the vector, since the selection procedure can result in isolation of cells that have incorrectly amplified the endogenous amplifiable marker(s) rather than the vector-encoded amplifiable marker(s). This approach is also useful to select against cells that develop resistance to the selective agent by mechanisms that do not involve gene amplification. The approach using two or more amplifiable markers is advantageous in these situations because the probability of a cell developing resistance to two or more selective agents (resistance to which is encoded by two or more amplifiable markers) without amplifying the integrated vector and flanking gene of interest is significantly lower than the probability of the cell developing resistance to any single selective agent. Thus, by selecting for two or more vector encoded amplifiable markers, either simultaneously or sequentially, a greater percentage of cells that are ultimately isolated will contain the amplified vector and gene of interest.

Thus, in another embodiment, the vectors may contain two or more (*i.e.,* two, three, four, five, or more, and most preferably two) amplifiable markers. This approach allows more efficient amplification of the vector sequences and adjacent gene of interest following activation of expression.

Examples ofamplifiable markers that may be used constructing the present vectors include, but are not limited to, dihydrofolate reductase, adenosine deaminase, aspartate transcarbamylase, dihydro-orotase, and carbamyl phosphate synthase.

It is also understood that any of the constructs described herein may contain a eukaryotic viral origin of replication, either in place of, or in conjunction with an amplifiable marker. The presence of the viral origin of replication allows the integrated vector and adjacent endogenous gene to be isolated as an episome and/or amplified to high copy number upon introduction of the appropriate viral replication protein. Examples of useful viral origins include, but are not limited to, SV40 ori and EBV ori P.

The constructs disclosed herein may consist essentially of the components specifically described for these constructs. It is also understood that the above constructs are examples of constructs useful in the methods described herein, but that functional equivalents of such constructs may be employed.

The term "vector" is understood to generally refer to the vehicle by which the nucleotide sequence is introduced into the cell. It is not intended to be limited to any specific sequence. The vector could itself be the nucleotide sequence that activates the endogenous gene or could contain the sequence that activates the endogenous gene. Thus, the vector could be simply a linear or circular polynucleotide containing essentially only those sequences necessary for activation, or could be these sequences in a larger polynucleotide or other construct such as a DNA or RNA viral genome, a whole virion, or other biological construct used to introduce the critical nucleotide sequences into a cell.

The vector can contain DNA sequences that exist in nature or that have been created by genetic engineering or synthetic processes.

The construct, upon nonhomologous integration into the genome of a cell, can activate expression of an endogenous gene. Expression of the endogenous gene may result in production of full length protein, or in production of a truncated biologically active form of the endogenous protein, depending on the integration site (e.g., upstream region versus intron 2). The activated gene may be a known gene (e.g., previously cloned or characterized) or unknown gene (previously not cloned or characterized). The function of the gene may be known or unknown.

Examples of proteins with known activities include, but are not limited to, cytokines, growth factors, neurotransmitters, enzymes, structural proteins, cell surface receptors, intracellular receptors, hormones, antibodies, and transcription factors. Specific examples of known proteins that can be produced by this method include, but are not limited to, erythropoietin, insulin, growth hormone, glucocerebrosidase, tissue plasminogen activator, granulocyte-colony stimulating factor (G-CSF), granulocyte/macrophage colony stimulating factor (GM-CSF), interferon α, interferon β, interferon γ, interleukin-2, interleukin-3, interleukin-4, interleukin-6, interleukin-8, interleukin-10, interleukin-11, interleukin-12, interleukin-13, interleukin-14, TGF-β, blood clotting factor V, blood clotting factor VII, blood clotting factor VIII, blood clotting factor IX, blood clotting factor X, TSH-β, bone growth factor-2, bone growth factor-7, tumor necrosis factor, alpha-1 antitrypsin, anti-thrombin III, leukemia inhibitory factor, glucagon, Protein C, protein kinase C, macrophage colony stimulating factor (M-CSF), stem cell factor, follicle stimulating hormone β, urokinase, nerve growth factors, insulin-like growth factors, insulinotropin, parathyroid hormone, lactoferrin, complement inhibitors, platelet derived growth factor, keratinocyte growth factor, hepatocyte growth factor, endothelial cell growth factor, neurotropin-3, thrombopoietin, chorionic gonadotropin, thrombomodulin, alpha glucosidase, epidermal growth factor, and fibroblast growth factor. The invention also allows the activation of a variety of genes expressing transmembrane proteins, and production and isolation of such proteins, including but not limited to cell surface receptors for growth factors, hormones, neurotransmitters and cytokines such as those described above, transmembrane ion channels, cholesterol receptors, receptors for lipoproteins (including LDLs and HDLs) and other lipid moieties, integrins and other extracellular matrix receptors, cytoskeletal anchoring proteins, immunoglobulin receptors, CD antigens (including CD2, CD3, CD4, CD8, and CD34 antigens), and other cell surface transmembrane structural and functional proteins that are known in the art. As one of ordinary skill will appreciate, other cellular proteins and receptors that are known in the art may also be produced by the methods of the invention.

One of the advantages of the method described herein is that virtually any gene can be activated. However, since genes have different genomic structures, including different intron/exon boundaries and locations of start codons, a variety of activation constructs is provided to activate the maximum number of different genes within a population of cells.

These constructs can be transfected separately into cells to produce libraries. Each library contains cells with a unique set of activated genes. Some genes will be activated by several different activation constructs. In addition, portions of a gene can be activated to produce truncated, biologically active proteins. Truncated proteins can be produced, for example, by integration of an activation construct into introns or exons in the middle of an endogenous gene rather than upstream of the second exon.

Use of different constructs also allows the activated gene to be modified to contain new sequences. For example, a secretion signal sequence can be included on the activation construct to facilitate the secretion of the activated gene. In some cases, depending on the intron/exon structure or the gene of interest, the secretion signal sequence can replace all or part of the signal sequence of the endogenous gene. In other cases, the signal sequence will allow a protein which is normally located intracellularly to be secreted.

The regulatory sequence on the vector can be a constitutive promoter. Alternatively, the promoter may be inducible. Use of inducible promoters will allow low basal levels of activated protein to be produced by the cell during routine culturing and expansion. The cells may then be induced to produce large amounts of the desired proteins, for example, during manufacturing or screening. Examples of inducible promoters include, but are not limited to, the tetracycline inducible promoter and the metallothionein promoter.

In preferred embodiments of the invention, the regulatory sequence on the vector may be a tissue specific promoter or an enhancer.

The regulatory sequence on the vector can be isolated from cellular or viral genomes. Examples of cellular regulatory sequences include, but are not limited to, regulatory elements from the actin gene, metallothionein I gene, immunoglobulin genes, casein I gene, serum albumin gene, collagen gene, globin genes, laminin gene, spectrin gene, ankyrin gene, sodium/potassium ATPase gene, and tubulin gene. Examples of viral regulatory sequences include, but are not limited to, regulatory elements from *Cytomegalovirus* (CMV) immediate early gene, adenovirus late genes, SV40 genes, retroviral LTRs, and *Herpesvirus* genes. Typically, regulatory sequences contain binding sites for transcription factors such as NF-kB, SP-1, TATA binding protein, AP-1, and CAAT binding protein. Functionally, the regulatory sequence is defined by its ability to promote, enhance, or otherwise alter transcription of an endogenous gene.

In preferred embodiments, the regulatory sequence is a viral promoter. In highly preferred embodiments, the promoter is the CMV immediate early gene promoter. In alternative embodiments, the regulatory element is a cellular, non-viral promoter.

In preferred embodiments, the regulatory element contains an enhancer. In highly preferred embodiments, the enhancer is the cytomegalovirus immediate early gene enhancer. In alternative embodiments, the enhancer is a cellular, non-viral enhancer.

The transcriptional regulatory sequence can be comprised of scaffold-attachment regions or matrix attachment sites, negative regulatory elements, and transcription factor binding sites. Regulatory sequences can also include locus control regions.

The invention encompasses the use of retrovirus transcriptional regulatory sequences, e.g., long terminal repeats. Where these are used, however, they are not necessarily linked to any retrovirus sequence that materially affects the function of the transcriptional regulatory sequence as a promoter or enhancer of transcription of the endogenous gene to be activated (i.e., the cellular gene with which the transcriptional regulatory sequence recombines to activate).

The construct may contain a regulatory sequence which is not operably linked to exonic sequences on the vector. For example, when the regulatory element is an enhancer, it can integrate near an endogenous gene (e.g., upstream, downstream, or in an intron) and stimulate expression of the gene from its endogenous promoter. By this mechanism of activation, exonic sequences from the vector are absent in the transcript of the activated gene.

Alternatively, the regulatory element may be operably linked to an exon. The exon may be a naturally occurring sequence or may be non-naturally occurring (e.g., produced synthetically). To activate endogenous genes lacking a start codon in their first exon (e.g., follicle stimulating hormone-β), a start codon is preferably omitted from the exon on the vector. To activate endogenous genes containing a start codon in the first exon (e.g., erythropoietin and growth hormone), the exon on the vector preferably contains a start codon, usually ATG and preferably an efficient translation initiation site (Kozak, J. Mol Biol. 196: 947 (1987)). The exon may contain additional codons following the start codon. These codons may be derived from a naturally occurring gene or may be non-naturally occurring (e.g., synthetic). The codons may be the same as the codons present in the first exon of the endogenous gene to be activated. Alternatively, the codons may be different than the codons present in the first exon of the endogenous gene. For example, the codons may encode an epitope tag, signal secretion sequence, transmembrane domain, selectable marker, or screenable marker. Optionally, an unpaired splice donor site may be present immediately 3' of the exonic sequence. When the structure of the gene to be activated is known, the splice donor site should be placed adjacent to the vector exon in a location such that the codons in the vector will be in frame with the codons of the second exon of the endogenous gene following splicing. When the structure of the endogenous gene to be activated is not known, separate constructs, each containing a different reading frame, are used.

Operably linked is defined as a configuration that allows transcription through the designated sequence(s). For example, a regulatory sequence that is operably linked to an exonic sequence indicates that the exonic sequence is transcribed. When a start codon is present on the vector, operably linked also indicates that the open reading frame from the vector exon is in frame with the open reading frame of the endogenous gene. Following nonhomologous integration, the regulatory sequence (e.g., a promoter) on the vector becomes operably linked to an endogenous gene and facilitates transcription initiation, at a site generally referred to as a CAP site. Transcription proceeds through the exonic elements on the vector (and, if present, through the start codon, open reading frame, and/or unpaired splice donor site), and through the endogenous gene. The primary transcript produced by this operable linkage is spliced to create a chimeric transcript containing exonic sequences from both the vector and the endogenous gene. This transcript is capable of producing the endogenous protein when translated.

An exon or "exonic sequence" is defined as any transcribed sequence that is present in the mature RNA molecule. The exon on the vector may contain untranslated sequences, for example, a 5' untranslated region. Alternatively, or in conjunction with the untranslated sequences, the exon may contain coding sequences such as a start codon and open reading frame. The open reading frame can encode naturally occurring amino acid sequences or non-naturally occurring amino acid sequences (e.g., synthetic codons). The open reading frame may also encode a signal secretion sequence, epitope tag, exon, selectable marker, screenable marker, or nucleotides that function to allow the open reading frame to be preserved when spliced to an endogenous gene.

Splicing of primary transcripts, the process by which introns are removed, is directed by a splice donor site and a splice acceptor site, located at the 5' and 3' ends of introns, respectively. The consensus sequence for splice donor sites is (A/C)AG GURAGU (where R represents a purine nucleotide) with nucleotides in positions 1-3 located in the exon and nucleotides GURAGU located in the intron.

An unpaired splice donor site is defined herein as a splice donor site present on the activation construct without a downstream splice acceptor site. When the vector is integrated by nonhomologous recombination into a host cell's genome, the unpaired splice donor site becomes paired with a splice acceptor site from an endogenous gene. The splice donor site from the vector, in conjunction with the splice acceptor site from the endogenous gene, will then direct the excision of all of the sequences between the vector splice donor site and the endogenous splice acceptor site. Excision of these intervening sequences removes sequences that interfere with translation of the endogenous protein.

The terms upstream and downstream, as used herein, are intended to mean in the 5' or in the 3' direction, respectively, relative to the coding strand. The term "upstream region" of a gene is defined as the nucleic acid sequence 5' of its second exon (relative to the coding strand) up to and including the last exon of the first adjacent gene having the same coding strand. Functionally, the upstream region is any site 5' of the second exon of an endogenous gene capable of allowing a nonhomologously integrated vector to become operably linked to the endogenous gene.

The vector construct can contain a selectable marker to facilitate the identification and isolation of cells containing a nonhomologously integrated activation construct. Examples of selectable markers include genes encoding neomycin resistance (neo), hypoxanthine phosphoribosyl transferase (HPRT), puromycin (pac), dihydro-orotase glutamine synthetase (GS), histidine D (his D), carbamyl phosphate synthase (CAD), dihyrofolate reductase (DHFR), multidrug resistance 1 (mdrl), aspartate transcarbamylase, xanthine-guanine phosphoribosyl transferase (gpt), and adenosine deaminase (ada).

Alternatively, the vector can contain a screenable marker, in place of or in addition to, the selectable marker. A screenable marker allows the cells containing the vector to be isolated without placing them under drug or other selective pressures. Examples of screenable markers include genes encoding cell surface proteins, fluorescent proteins, and enzymes. The vector containing cells may be isolated, for example, by FACS using fluorescently-tagged antibodies to the cell surface protein or substrates that can be converted to fluorescent products by a vector encoded enzyme.

Alternatively, selection can be effected by phenotypic selection for a trait provided by the endogenous gene product. The activation construct, therefore, can lack a selectable marker other than the "marker" provided by the endogenous gene itself In this embodiment, activated cells can be selected based on a phenotype conferred by the activated gene. Examples of selectable phenotypes include cellular proliferation, growth factor independent growth, colony formation, cellular differentiation (e.g., differentiation into a neuronal cell, muscle cell, epithelial cell, etc.), anchorage independent growth, activation of cellular factors (e.g., kinases, transcription factors, nucleases, etc.), expression of cell surface receptors/proteins, gain or loss of cell-cell adhesion, migration, and cellular activation (e.g., resting versus activated T cells).

A selectable marker may also be omitted from the construct when transfected cells are screened for gene activation products without selecting for the stable integrants. This is particularly useful when the efficiency of stable integration is high.

The vector may contain one or more (i.e., one, two, three, four, five, or more, and most preferably one or two) amplifiable markers to allow for selection of cells containing increased copies of the integrated vector and the adjacent activated endogenous gene. Examples of amplifiable markers include but are not limited to dihydrofolate reductase (DHFR), adenosine deaminase (ada), dihydro-orotase glutamine synthetase (GS), and carbamyl phosphate synthase (CAD).

The vector may contain eukaryotic viral origins of replication useful for gene amplification. These origins may be present in place of, or in conjunction with, an amplifiable marker.

The vector may also contain genetic elements useful for the propagation of the construct in micro-organisms. Examples of useful genetic elements include microbial origins of replication and antibiotic resistance markers.

These vectors, and any of the vectors disclosed herein, and obvious variants recognized by one of ordinary skill in the art, can be used in any of the methods described above to form any of the compositions producible by those methods.

Nonhomologous integration of the construct into the genome of a cell results in the operable linkage between the regulatory elements from the vector and the exons from an endogenous gene. In preferred embodiments, the insertion of the vector regulatory sequences is used to upregulate expression of the endogenous gene. Upregulation of gene expression includes converting a transcriptionally silent gene to a transcriptionally active gene. It also includes enhancement of gene expression for genes that are already transcriptionally active, but produce protein at levels lower than desired. In other embodiments, expression of the endogenous gene may be affected in other ways such as creation of an inducible phenotype, or changing the tissue specificity of expression.

*In vitro* methods of production of a gene expression product may comprise, for example, (a) introducing a vector of the invention into a cell; (b) allowing the vector to integrate into the genome of the cell by non-homologous recombination; (c) allowing over-expression of an endogenous gene in the cell by upregulation of the gene by the transcriptional regulatory sequence contained on the vector; (d) screening the cell for over-expression of the endogenous gene; and (e) culturing the cell under conditions favoring the production of the expression product of the endogenous gene by the cell. Such *in vitro* methods of the invention may further comprise isolating the expression product to produce an isolated gene expression product. In such methods, any art-known method of protein isolation may be advantageously used, including but not limited to chromatography (*e.g*., HPLC, FPLC, LC, ion exchange, affinity, size exclusion, and the like), precipitation (*e.g*., ammonium sulfate precipitation, immunoprecipitation, and the like), electrophoresis, and other methods of protein isolation and purification that will be familiar to one of ordinary skill in the art.

Analogously, *in vivo* methods ofproduction of a gene expression product may comprise, for example, (a) introducing a vector of the invention into a cell; (b) allowing the vector to integrate into the genome of the cell by non-homologous recombination; (c) allowing over-expression of an endogenous gene in the cell by upregulation of the gene by the transcriptional regulatory sequence contained on the vector; (d) screening the cell for over-expression of the endogenous gene; and (e) introducing the isolated and cloned cell into a eukaryote under conditions favoring the overexpression of the endogenous gene by the cell *in vivo* in the eukaryote. Any eukaryote may be advantageously used, including fungi (particularly yeasts), plants, and animals, more preferably animals, still more preferably vertebrates, and most preferably mammals, particularly humans. In certain related instances, also referred to are such methods which further comprise isolating and cloning the cell prior to introducing it into the eukaryote.

As used herein the phrases "conditions favoring the production" of an expression product, "conditions favoring the overexpression" of a gene, and "conditions favoring the activation" of a gene, in a cell or by a cell *in vitro* refer to any and all suitable environmental, physical, nutritional or biochemical parameters that allow, facilitate, or promote production ofan expression product, or overexpression or activation of a gene, by a cell *in vitro.* Such conditions may, of course, include the use of culture media, incubation, lighting, humidity, etc., that are optimal or that allow, facilitate, or promote production of an expression product, or overexpression or activation of a gene, by a cell *in vitro.* Analogously, as used herein the phrases "conditions favoring the production" of an expression product, "conditions favoring the overexpression" of a gene, and "conditions favoring the activation" of a gene, in a cell or by a cell *in vivo* refer to any and all suitable environmental, physical, nutritional, biochemical, behavioral, genetic, and emotional parameters under which an animal containing a cell is maintained, that allow, facilitate, or promote production of an expression product, or overexpression or activation of a gene, by a cell in a eukaryote *in vivo.* Whether a given set of conditions are favorable for gene expression, activation, or overexpression, *in vitro* or *in vivo,* may be determined by one of ordinary skill using the screening methods described and exemplified below, or other methods for measuring gene expression, activation, or overexpression that are routine in the art.

Also referred to are cells made by any of the above methods. Also referred to are cells containing the vector constructs, cells in which the vector constructs have integrated, and cells which are over-expressing desired gene products from an endogenous gene, over-expression being driven by the introduced transcriptional regulatory sequence.

Cells used in this invention can be derived from any eukaryotic species and can be primary, secondary, or immortalized. Furthermore, the cells can be derived from any tissue in the organism. Examples of useful tissues from which cells can be isolated and activated include, but are not limited to, liver, kidney, spleen, bone marrow, thymus, heart, muscle, lung, brain, testes, ovary, islet, intestinal, bone marrow, skin, bone, gall bladder, prostate, bladder, embryos, and the immune and hematopoietic systems. Cell types include fibroblast, epithelial, neuronal, stem, and follicular. However, any cell or cell type can be used to activate gene expression using this invention.

The methods can be carried out in any cell of eukaryotic origin, such as fungal, plant or animal. Preferred embodiments include vertebrates and particularly mammals, and more particularly, humans.

The construct can be integrated into primary, secondary, or immortalized cells. Primary cells are cells that have been isolated from a vertebrate and have not been passaged. Secondary cells are primary cells that have been passaged, but are not immortalized. Immortalized cells are cell lines that can be passaged, apparently indefinitely.

In preferred embodiments, the cells are immortalized cell lines. Examples of immortalized cell lines include, but are not limited to, HT1080, HeLa, Jurkat, 293 cells, KB carcinoma, T84 colonic epithelial cell line, Raji, Hep G2 or Hep 3B hepatoma cell lines, A2058 melanoma, U937 lymphoma, and WI38 fibroblast cell line, somatic cell hybrids, and hybridomas.

Cells used in this invention can be derived from any eukaryotic species, including but not limited to mammalian cells (such as rat, mouse, bovine, porcine, sheep, goat, and human), avian cells, fish cells, amphibian cells, reptilian cells, plant cells, and yeast cells. Preferably, overexpression of an endogenous gene or gene product from a particular species is accomplished by activating gene expression in a cell from that species. For example, to overexpress endogenous human proteins, human cells are used. Similarly, to overexpress endogenous bovine proteins, for example bovine growth hormone, bovine cells are used.

The cells can be derived from any tissue in the eukaryotic organism. Examples of useful vertebrate tissues from which cells can be isolated and activated include, but are not limited to, liver, kidney, spleen, bone marrow, thymus, heart, muscle, lung, brain, immune system (including lymphatic), testes, ovary, islet, intestinal, stomach, bone marrow, skin, bone, gall bladder, prostate, bladder, zygotes, embryos, and hematopoietic tissue. Useful vertebrate cell types include, but are not limited to, fibroblasts, epithelial cells, neuronal cells, germ cells (*i.e.,* spermatocytes/spermatozoa and oocytes), stem cells, and follicular cells. Examples of plant tissues from which cells can be isolated and activated include, but are not limited to, leaf tissue, ovary tissue, stamen tissue, pistil tissue, root tissue, tubers, gametes, seeds, embryos, and the like. One of ordinary skill will appreciate, however, that any eukaryotic cell or cell type can be used to activate gene expression using the present invention.

Any of the cells produced by any of the methods described are useful for screening for expression of a desired gene product and for providing desired amounts of a gene product that is over-expressed in the cell. The cells can be isolated and cloned.

Cells produced by this method can be used to produce protein *in vitro* (e.g., for use as a protein therapeutic) or *in vivo* (e.g., for use in cell therapy).

Commercial growth and production conditions often vary from the conditions used to grow and prepare cells for analytical use (e.g., cloning, protein or nucleic acid sequencing, raising antibodies, X-ray crystallography analysis, enzymatic analysis, and the like). Scale up of cells for growth in roller bottles involves increase in the surface area on which cells can attach. Microcarrier beads are, therefore, often added to increase the surface area for commercial growth. Scale up of cells in spinner culture may involve large increases in volume. Five liters or greater can be required for both microcarrier and spinner growth. Depending on the inherent potency (specific activity) of the protein of interest, the volume can be as low as 1-10 liters. 10-15 liters is more common. However, up to 50-100 liters may be necessary and volume can be as high as 10,000-15,000 liters. In some cases, higher volumes may be required. Cells can also be grown in large numbers of T flasks, for example 50-100.

Despite growth conditions, protein purification on a commercial scale can also vary considerably from purification for analytic purposes. Protein purification in a commercial practical context can be initially the mass equivalent of 10 liters of cells at approximately 10⁴ cells/ml. Cell mass equivalent to begin protein purification can also be as high as 10 liters of cells at up to 10⁶ or 10⁷ cells/ml. As one of ordinary skill will appreciate, however, a higher or lower initial cell mass equivalent may also be advantageously used in the present methods.

Another commercial growth condition, especially when the ultimate product is used clinically, is cell growth in serum-free medium, by which is intended medium containing no serum or not in amounts that are required for cell growth. This obviously avoids the undesired co-purification oftoxic contaminants (e.g., viruses) or other types of contaminants, for example, proteins that would complicate purification. Serum-free media for growth of cells, commercial sources for such media, and methods for cultivation of cells in serum-free media, are well-known to those of ordinary skill in the art.

A single cell made by the methods described above can over-express a single gene or more than one gene. More than one gene can be activated by the integration of a single construct or by the integration of multiple constructs in the same cell (i.e., more than one type of construct). Therefore, a cell can contain only one type of vector construct or different types of constructs, each capable of activating an endogenous gene.

The invention is also directed to methods for making the cells described above by one or more of the following: introducing one or more of the vector constructs; allowing the introduced construct(s) to integrate into the genome of the cell by non-homologous recombination; allowing over-expression of one or more endogenous genes in the cell; and isolating and cloning the cell.

The term "transfection" has been used herein for convenience when discussing introducing a polynucleotide into a cell. However, it is to be understood that the specific use of this term has been applied to generally refer to the *introduction* of the polynucleotide into a cell and is also intended to refer to the introduction by other methods described herein such as electroporation, liposome-mediated introduction, retrovirus-mediated introduction, and the like (as well as according to its own specific meaning).

The vector can be introduced into the cell by a number of methods known in the art. These include, but are not limited to, electroporation, calcium phosphate precipitation, DEAE dextran, lipofection, and receptor mediated endocytosis, polybrene, particle bombardment, and microinjection. Alternatively, the vector can be delivered to the cell as a viral particle (either replication competent or deficient). Examples of viruses useful for the delivery of nucleic acid include, but are not limited to, adenoviruses, adeno-associated viruses, retroviruses, Herpesviruseses, and vaccinia viruses. Other viruses suitable for delivery of nucleic acid molecules into cells that are known to one of ordinary skill may be equivalently used in the present methods.

Following transfection, the cells are cultured under conditions, as known in the art, suitable for nonhomologous integration between the vector and the host cell's genome. Cells containing the nonhomologously integrated vector can be further cultured under conditions, as known in the art, allowing expression of activated endogenous genes.

The vector construct can be introduced into cells on a single DNA construct or on separate constructs and allowed to concatemerize.

Whereas in preferred embodiments, the vector construct is a double-stranded DNA vector construct, vector constructs also include single-stranded DNA, combinations of single- and double-stranded DNA, single-stranded RNA, double-stranded RNA, and combinations of single- and double-stranded RNA. Thus, for example, the vector construct could be single-stranded RNA which is converted to cDNA by reverse transcriptase, the cDNA converted to double-stranded DNA, and the double-stranded DNA ultimately recombining with the host cell genome.

In preferred embodiments, the constructs are linearized prior to introduction into the cell. Linearization of the activation construct creates free DNA ends capable of reacting with chromosomal ends during the integration process. In general, the construct is linearized downstream of the regulatory element (and exonic and splice donor sequences, if present). Linearization can be facilitated by, for example, placing a unique restriction site downstream of the regulatory sequences and treating the construct with the corresponding restriction enzyme prior to transfection. While not required, it is advantageous to place a "spacer" sequence between the linearization site and the proximal most functional element (e.g., the unpaired splice donor site) on the construct. When present, the spacer sequence protects the important functional elements on the vector from exonucleolytic degradation during the transfection process. The spacer can be composed of any nucleotide sequence that does not change the essential functions of the vector as described herein.

Circular constructs can also be used to activate endogenous gene expression. It is known in the art that circular plasmids, upon transfection into cells, can integrate into the host cell genome. Presumably, DNA breaks occur in the circular plasmid during the transfection process, thereby generating free DNA ends capable of joining to chromosome ends. Some of these breaks in the construct will occur in a location that does not destroy essential vector functions (e.g., the break will occur downstream of the regulatory sequence), and therefore, will allow the construct to be integrated into a chromosome in a configuration capable of activating an endogenous gene. As described above, spacer sequences may be placed on the construct (e.g., downstream of the regulatory sequences). During transfection, breaks that occur in the spacer region will create free ends at a site in the construct suitable for activation of an endogenous gene following integration into the host cell genome.

Also referred to are libraries of cells made by the above described methods. A library can encompass all of the clones from a single transfection experiment or a subset of clones from a single transfection experiment. The subset can over-express the same gene or more than one gene, for example, a class of genes. The transfection can have been done with a single type of construct or with more than one type of construct.

A library can also be formed by combining all of the recombinant cells from two or more transfection experiments, by combining one or more subsets of cells from a single transfection experiment or by combining subsets of cells from separate transfection experiments. The resulting library can express the same gene, or more than one gene, for example, a class of genes. Again, in each of these individual transfections, a unique construct or more than one construct can be used.

Libraries can be formed from the same cell type or different cell types. The library can be composed of a single type of cell containing a single type of activation construct which has been integrated into chromosomes at spontaneous DNA breaks or at breaks generated by radiation, restriction enzymes, and/or DNA breaking agents, applied either together (to the same cells) or separately (applied to individual groups of cells and then combining the cells together to produce the library). The library can be composed of multiple types of cells containing a single or multiple constructs which were integrated into the genome of a cell treated with radiation, restriction enzymes, and/or DNA breaking agents, applied either together (to the same cells) or separately (applied to individual groups of cells and then combining the cells together to produce the library).

Also referred to are methods for making libraries by selecting various subsets of cells from the same or different transfection experiments. For example, all of the cells expressing nuclear factors (as determined by the presence of nuclear green fluorescent protein in cells transfected with construct 20) can be pooled to create a library of cells with activated nuclear factors. Similarly, cells expressing membrane or secreted proteins can be pooled. Cells can also be grouped by phenotype, for example, growth factor independent growth, growth factor independent proliferation, colony formation, cellular differentiation (e.g., differentiation into a neuronal cell, muscle cell, epithelial cell, etc.), anchorage independent growth, activation of cellular factors (e.g., kinases, transcription factors, nucleases, etc.), gain or loss of cell-cell adhesion, migration, or cellular activation (e.g., resting versus activated T cells).

Also referred to are methods of using libraries of cells to over-express an endogenous gene. The library is screened for the expression of the gene and cells are selected that express the desired gene product. The cell can then be used to purify the gene product for subsequent use. Expression of the cell can occur by culturing the cell *in vitro* or by allowing the cell to express the gene *in vivo*.

Also referred to are methods of using libraries to identify novel gene and gene products.

Also referred to are methods for increasing the efficiency of gene activation by treating the cells with agents that stimulate or effect the patterns of non-homologous integration. It has been demonstrated that gene expression patterns, chromatin structure, and methylation patterns can differ dramatically from cell type to cell type. Even different cell lines from the same cell type can have significant differences. These differences can impact the patterns of non-homologous integration by affecting both the DNA breakage pattern and the repair process. For example, chromatinized stretches of DNA (characteristics likely associated with inactive genes) may be more resistant to breakage by restriction enzymes and chemical agents, whereas they may be susceptible to breakage by radiation.

Furthermore, inactive genes can be methylated. In this case, restriction enzymes that are blocked by CpG methylation will be unable to cleave methylated sites near the inactive gene, making it more difficult to activate that gene using methylation-sensitive enzymes. These problems can be circumvented by creating activation libraries in several cell lines using a variety of DNA breakage agents. By doing this, a more complete integration pattern can be created and the probability of activating a given gene maximized.

The methods of the invention can include introducing double strand breaks into the DNA of the cell containing the endogenous gene to be over-expressed. These methods introduce double-strand breaks into the genomic DNA in the cell prior to or simultaneously with vector integration. The mechanism of DNA breakage can have a significant effect on the pattern of DNA breaks in the genome. As a result, DNA breaks produced spontaneously or artificially with radiation, restriction enzymes, bleomycin, or other breaking agents, can occur in different locations.

In order to increase integration efficiency and to improve the random distribution of integration sites, cells can be treated with low, intermediate, or high doses of radiation prior to or following transfection. By artificially inducing double strand breaks, the transfected DNA can now integrate into the host cell chromosome as part of the DNA repair process. Normally, creation of double strand breaks to serve as the site of integration is the rate limiting step. Thus, by increasing chromosome breaks using radiation (or other DNA damaging agents), a larger number of integrants can be obtained in a given transfection. Furthermore, the mechanism of DNA breakage by radiation is different than by spontaneous breakage.

Radiation can induce DNA breaks directly when a high energy photon hits the DNA molecule. Alternatively, radiation can activate compounds in the cell which in turn, react with and break the DNA strand. Spontaneous breaks, on the other hand, are thought to occur by the interaction between reactive compounds produced in the cell (such as superoxides and peroxides) and the DNA molecule. However, DNA in the cell is not present as a naked, deproteinized polymer, but instead is bound to chromatin and present in a condensed state. As a result, some regions are not accessible to agents in the cell that cause double strand breaks. The photons produced by radiation have wave lengths short enough to hit highly condensed regions of DNA, thereby inducing breaks in DNA regions that are under represented in spontaneous breaks. Thus, radiation is capable of creating different DNA breakage patterns, which in turn, should lead to different integration patterns.

As a result, libraries produced using the same activation construct in cells with and without radiation treatment will potentially contain different sets of activated genes. Finally, radiation treatment increases efficiency of nonhomologous integration by up to 5-10 fold, allowing complete libraries to be created using fewer cells. Thus, radiation treatment increases the efficiency of gene activation and generates new integration and activation patterns in transfected cells. Useful types of radiation include α, β, γ, x-ray, and ultraviolet radiation. Useful doses of radiation vary for different cell types, but in general, dose ranges resulting in cell viabilities of 0.1% to >99% are useful. For HT1080 cells, this corresponds to radiation doses from a ¹³⁷Cs source of approximately 0.1 rads to 1000 rads. Other doses may also be useful as long as the dose either increases the integration frequency or changes the pattern of integration sites.

In addition to radiation, restriction enzymes can be used to artificially induce chromosome breaks in transfected cells. As with radiation, DNA restriction enzymes can create chromosome breaks which, in turn, serve as integration sites for the transfected DNA. This larger number of DNA breaks increases the overall efficiency of integration of the activation construct. Furthermore, the mechanism of breakage by restriction enzymes differs from that by radiation, the pattern of chromosome breaks is also likely to be different.

Restriction enzymes are relatively large molecules compared to photons and small metabolites capable of damaging DNA. As a result, restriction enzymes will tend to break regions that are less condensed then the genome as a whole. If the gene of interest lies within an accessible region of the genome, then treatment of the cells with a restriction enzyme can increase the probability of integrating the activation construct upstream of the gene of interest. Since restriction enzymes recognize specific sequences, and since a given restriction site may not lie upstream of the gene of interest, a variety of restriction enzymes can be used. It may also be important to use a variety of restriction enzymes since each enzyme has different properties (e.g., size, stability, ability to cleave methylated sites, and optimal reaction conditions) that affect which sites in the host chromosome will be cleaved. Each enzyme, due to the different distribution of cleavable restriction sites, will create a different integration pattern.

Therefore, introduction of restriction enzymes (or plasmids capable of expressing restriction enzymes) before, during, or after introduction of the activation construct will result in the activation of different sets of genes. Finally, restriction enzyme-induced breaks increase the integration efficiency by up to 5-10 fold (Yorifuji et al., Mut. Res. 243:121 (1990)), allowing fewer cells to be transfected to produce a complete library. Thus, restriction enzymes can be used to create new integration patterns, allowing activation of genes which failed to be activated in libraries produced by non-homologous recombination at spontaneous breaks or at other artificially induced breaks.

Restriction enzymes can also be used to bias integration of the activation construct to a desired site in the genome. For example, several rare restriction enzymes have been described which cleave eukaryotic DNA every 50-1000 kilobases, on average. If a rare restriction recognition sequence happens to be located upstream of a gene of interest, by introducing the restriction enzyme at the time of transfection along with the activation construct, DNA breaks can be preferentially upstream of the gene of interest. These breaks can then serve as sites for integration of the activation construct. Any enzyme can be that cleaves in an appropriate location in or near the gene of interest and its site is under-represented in the rest of the genome or its site is over-represented near genes (e.g., restriction sites containing CpG). For genes that have not been previously identified, restriction enzymes with 8 bp recognition sites (e.g., *Not*I, *Sfi*I, *Pme*I, *Swa*I, *Sse*I, *Srf*I, *Sgr*A1, *Pac*I, *Asc*I, SgfI, and *Sse8387I*), enzymes recognizing CpG containing sites (e.g., *Eag*I, *Bsi-WI, Mlu*I, and *Bss*HII) and other rare cutting enzymes can be used.

In this way, "biased" libraries can be created which are enriched for certain types of activated genes. In this respect, restriction enzyme sites containing CpG dinucleotides are particularly useful since these sites are under-represented in the genome at large, but over-represented in the form of CpG islands at the 5' end of many genes, the very location that is useful for gene activation. Enzymes recognizing these sites, therefore, will preferentially cleave at the 5' end of genic sequences.

Restriction enzymes can be introduced into the host cell by several methods. First, restriction enzymes can be introduced into the cell by electroporation (Yorifuji et al., Mut. Res. 243:121 (1990); Winegar et al., Mut. Res. 225:49 (1989)). In general, the amount of restriction enzyme introduced into the cell is proportional to its concentration in the electroporation media. The pulse conditions must be optimized for each cell line by adjusting the voltage, capacitance, and resistance. Second, the restriction enzyme can be expressed transiently from a plasmid encoding the enzyme under the control of eukaryotic regulatory elements. The level of enzyme produced can be controlled by using inducible promoters, and varying the strength of induction. In some cases, it may be desirable to limit the amount of restriction enzyme produced (due to its toxicity). In these cases, weak or mutant promoters, splice sites, translation start codons, and poly A tails can be utilized to lower the amount of restriction enzyme produced. Third, restriction enzymes can be introduced by agents that fuse with or permeabilize the cell membrane. Liposomes and streptolysin O (Pimplikar et al.. J. Cell Biol. 125:1025 (1994)) are examples of this type of agent. Finally, mechanical perforation (Beckers et al., Cell 50:523-534 (1987)) and microinjection can also be used to introduce nucleases and other proteins into cells. However, any method capable of delivering active enzymes to a living cell is suitable.

DNA breaks induced by bleomycin and other DNA damaging agents can also produce DNA breakage patterns that are different. Thus, any agent or incubation condition capable of generating double strand breaks in cells is useful for increasing the efficiency and/or altering the sites of non-homologous recombination. Examples of classes of chemical DNA breaking agents include, but are not limited to, peroxides and other free radical generating compounds, alkylating agents, topoisomerase inhibitors, anti-neoplastic drugs, acids, substituted nucleotides, and enediyne antibiotics.

Specific chemical DNA breaking agents include, but are not limited to, bleomycin, hydrogen peroxide, cumene hydroperoxide, tert-butyl hydroperoxide, hypochlorous acid (reacted with aniline, 1-naphthylamine or 1-naphthol), nitric acid, phosphoric acid, doxorubicin, 9-deoxydoxorubicin, demethyl-6-deoxyrubicin, 5-iminodaunorubicin, adriamycin, 4'-(9- acridinylamino)methanesulfon-m-anisidide, neocarzinostatin, 8-methoxycaffeine, etoposide, ellipticine, iododeoxyuridine, and bromodeoxyuridine.

It has been shown that DNA repair machinery in the cell can be induced by pre-exposing the cell to low doses of a DNA breaking agent such as radiation or bleomycin. By pretreating cells with these agents approximately 24 hours prior to transfection, the cell will be more efficient at repairing DNA breaks and integrating DNA following transfection. In addition, higher doses of radiation or other DNA breaking agents can be used since the LD50 (the dose that results in lethality in 50% of the exposed cells) is higher following pretreatment. This allows random activation libraries to be created at multiple doses and results in a different distribution of integration sites within the host cell's chromosomes.

### Screening

Once an activation library (or libraries) is created, it can be screened using a number of assays. Depending on the characteristics of the protein(s) of interest (e.g., secreted versus intracellular proteins) and the nature of the activation construct used to create the library, any or all of the assays described below can be utilized. Other assay formats can also be used.

***ELISA***. Activated proteins can be detected using the enzyme-linked immunosorbent assay (ELISA). If the activated gene product is secreted, culture supernatants from pools of activation library cells are incubated in wells containing bound antibody specific for the protein of interest. If a cell or group of cells has activated the gene of interest, then the protein will be secreted into the culture media. By screening pools of library clones (the pools can be from 1 to greater than 100,000 library members), pools containing a cell(s) that has activated the gene of interest can be identified. The cell of interest can then be purified away from the other library members by sib selection, limiting dilution, or other techniques known in the art. In addition to secreted proteins, ELISA can be used to screen for cells expressing intracellular and membrane-bound proteins. In these cases, instead of screening culture supernatants, a small number of cells is removed from the library pool (each cell is represented at least 100-1000 times in each pool), lysed, clarified, and added to the antibody-coated wells.

***ELISA Spot Assay**.* ELISA spot are coated with antibodies specific for the protein of interest. Following coating, the wells are blocked with 1% BSA/PBS for 1 hour at 37°C. Following blocking, 100,000 to 500,000 cells from the random activation library are applied to each well (representing ~10% of the total pool). In general, one pool is applied to each well. If the frequency of a cell expressing the protein of interest is 1 in 10,000 (i.e., the pool consists of 10,000 individual clones, one of which expresses the protein of interest), then plating 500,000 cells per well will yield 50 specific cells. Cells are incubated in the wells at 37°C for 24 to 48 hours without being moved or disturbed. At the end of the incubation, the cells are removed and the plate is washed 3 times with PBS/0.05% Tween 20 and 3 times with PBS/1%BSA. Secondary antibodies are applied to the wells at the appropriate concentration and incubated for 2 hours at room temperature or 16 hours at 4°C. These antibodies can be biotinylated or labeled directly with horseradish peroxidase (HRP). The secondary antibodies are removed and the plate is washed with PBS/1% BSA. The tertiary antibody or streptavidin labeled with HRP is added and incubated for 1 hour at room temperature.

***FACS assay**.* The fluorescence-activated cell sorter (FACS) can be used to screen the random activation library in a number of ways. If the gene of interest encodes a cell surface protein, then fluorescently-labeled antibodies are incubated with cells from the activation library. If the gene of interest encodes a secreted protein, then cells can be biotinylated and incubated with streptavidin conjugated to an antibody specific to the protein of interest (Manz et al., Proc. Natl. Acad Sci. (USA) 92:1921 (1995)). Following incubation, the cells are placed in a high concentration of gelatin (or other polymer such as agarose or methylcellulose) to limit diffusion of the secreted protein. As protein is secreted by the cell, it is captured by the antibody bound to the cell surface. The presence of the protein of interest is then detected by a second antibody which is fluorescently labeled. For both secreted and membrane bound proteins, the cells can then be sorted according to their fluorescence signal. Fluorescent cells can then be isolated, expanded, and further enriched by FACS, limiting dilution, or other cell purification techniques known in the art.

***Magnetic Bead Separation.*** The principle of this technique is similar to FACS. Membrane bound proteins and captured secreted proteins (as described above) are detected by incubating the activation library with an antibody-conjugated magnetic beads that are specific for the protein of interest. If the protein is present on the surface of a cell, the magnetic beads will bind to that cell. Using a magnet, the cells expressing the protein of interest can be purified away from the other cells in the library. The cells are then released from the beads, expanded, analyzed, and further purified if necessary.

***RT-PCR**.* A small number of cells (equivalent to at least the number of individual clones in the pool) is harvested and lysed to allow purification of the RNA. Following isolation, the RNA is reversed-transcribed using reverse transcriptase. PCR is then carried out using primers specific for the cDNA of the gene of interest.

Alternatively, primers can be used that span the synthetic exon in the activation construct and the exon of the endogenous gene. This primer will not hybridize to and amplify the endogenously expressed gene ofinterest. Conversely, if the activation construct has integrated upstream of the gene of interest and activated gene expression, then this primer, in conjunction with a second primer specific for the gene will amplify the activated gene by virtue of the presence of the synthetic exon spliced onto the exon from the endogenous gene. Thus, this method can be used to detect activated genes in cells that normally express the gene of interest at lower than desired levels.

***Phenotypic Section**.* In this embodiment, cells can be selected based on a phenotype conferred by the activated gene. Examples of phenotypes that can be selected for include proliferation, growth factor independent growth, colony formation, cellular differentiation (e.g., differentiation into a neuronal cell, muscle cell, epithelial cell, etc.), anchorage independent growth, activation of cellular factors (e.g., kinases, transcription factors, nucleases, etc.), gain or loss of cell-cell adhesion, migration, and cellular activation (e.g., resting versus activated T cells). Isolation of activated cells demonstrating a phenotype, such as those described above, is important because the activation of an endogenous gene by the integrated construct is presumably responsible for the observed cellular phenotype. Thus, the activated gene may be an important therapeutic drug or drug target for treating or inducing the observed phenotype.

The sensitivity of each of the above assays can be effectively increased by transiently upregulating gene expression in the library cells. This can be accomplished for NF-κB site-containing promoters (on the activation construct) by adding PMA and tumor necrosis factor-α, *e.g*., to the library. Separately, or in conjunction with PMA and TNF-α, sodium butyrate can be added to further enhance gene expression. Addition of these reagents can increase expression of the protein of interest, thereby allowing a lower sensitivity assay to be used to identify the gene activated cell of interest.

Since large activation libraries are created to maximize activation of many genes, it is advantageous to organize the library clones in pools. Each pool can consist of 1 to greater than 100,000 individual clones. Thus, in a given pool, many activated proteins are produced, often in dilute concentrations (due to the overall size of the pool and the limited number of cells within the pool that produce a given activated protein). Thus, concentration of the proteins prior to screening effectively increases the ability to detect the activated proteins in the screening assay. One particularly useful method of concentration is ultrafiltration; however, other methods can also be used. For example, proteins can be concentrated non-specifically, or semi-specifically by adsorption onto ion exchange, hydrophobic, dye, hydroxyapatite, lectin, and other suitable resins under conditions that bind most or all proteins present. The bound proteins can then be removed in a small volume prior to screening. It is advantageous to grow the cells in serum free media to facilitate the concentration of proteins.

In another embodiment, a useful sequence that can be included on the activation construct is an epitope tag. The epitope tag can consist of an amino acid sequence that allows affinity purification of the activated protein (e.g., on immunoaffinity or chelating matrices). Thus, by including an epitope tag on the activation construct, all of the activated proteins from an activation library can be purified. By purifying the activated proteins away from other cellular and media proteins, screening for novel proteins and enzyme activities can be facilitated. In some instances, it may be desirable to remove the epitope tag following purification of the activated protein. This can be accomplished by including a protease recognition sequence (e.g., Factor IIa or enterokinase cleavage site) downstream from the epitope tag on the activation construct. Incubation of the purified, activated protein(s) with the appropriate protease will release the epitope tag from the proteins(s).

In libraries in which an epitope tag sequence is located on the activation construct, all of the activated proteins can be purified away from all other cellular and media proteins using affinity purification. This not only concentrates the activated proteins, but also purifies them away from other activities that can interfere with the assay used to screen the library.

Once a pool of clones containing cells over-expressing the gene of interest is identified, steps can be taken to isolate the activated cell. Isolation of the activated cell can be accomplished by a variety of methods known in the art. Examples of cell purification methods include limiting dilution, fluorescence activated cell sorting, magnetic bead separation, sib selection, and single colony purification using cloning rings.

In preferred embodiments of the invention, the methods include a process wherein the expression product is purified. In highly preferred embodiments, the cells expressing the endogenous gene product are cultured so as to produce amounts of gene product feasible for commercial application, and especially diagnostic and therapeutic and drug discovery uses.

Any vector used in the methods described herein can include an amplifiable marker. Thereby, amplification of both the vector and the DNA of interest (i.e., containing the over-expressed gene) occurs in the cell, and further enhanced expression ofthe endogenous gene is obtained. Accordingly, methods can include a step in which the endogenous gene is amplified.

Once the activated cell has been isolated, expression can be further increased by amplifying the locus containing both the gene of interest and the activation construct. This can be accomplished by each of the methods described below, either separately or in combination.

Amplifiable markers are genes that can be selected for higher copy number. Examples of amplifiable markers include dihydrofolate reductase, adenosine deaminase, aspartate transcarbamylase, dihydro-orotase, and carbamyl phosphate synthase. For these examples, the elevated copy number ofthe amplifiable marker and flanking sequences (including the gene of interest) can be selected for using a drug or toxic metabolite which is acted upon by the amplifiable marker. In general, as the drug or toxic metabolite concentration increases, cells containing fewer copies of the amplifiable marker die, whereas cells containing increased copies of the marker survive and form colonies. These colonies can be isolated, expanded, and analyzed for increased levels of production of the gene of interest.

Placement of an amplifiable marker on the activation construct results in the juxtaposition of the gene of interest and the amplifiable marker in the activated cell. Selection for activated cells containing increased copy number of the amplifiable marker and gene of interest can be achieved by growing the cells in the presence of increasing amounts of selective agent (usually a drug or metabolite). For example, amplification of dihydrofolate reductase (DHFR) can be selected using methotrexate.

As drug-resistant colonies arise at each increasing drug concentration, individual colonies can be selected and characterized for copy number of the amplifiable marker and gene of interest, and analyzed for expression of the gene of interest. Individual colonies with the highest levels of activated gene expression can be selected for further amplification in higher drug concentrations. At the highest drug concentrations, the clones will express greatly increased amounts of the protein of interest.

When amplifying DHFR, it is convenient to plate approximately 1 x 10⁷ cells at several different concentrations of methotrexate. Useful initial concentrations of methotrexate range from approximately 5 nM to 100 nM. However, the optimal concentration of methotrexate must be determined empirically for each cell line and integration site. Following growth in methotrexate containing media, colonies from the highest concentration of methotrexate are picked and analyzed for increased expression of the gene of interest. The clone(s) with the highest concentration of methotrexate are then grown in higher concentrations of methotrexate to select for further amplification of DHFR and the gene of interest. Methotrexate concentrations in the micromolar and millimolar range can be used for clones containing the highest degree of gene amplification.

Placement of a viral origin of replication(s) (e.g., ori P or SV40 in human cells, and polyoma ori in mouse cells) on the activation construct will result in the juxtaposition of the gene of interest and the viral origin of replication in the activated cell. The origin and flanking sequences can then be amplified by introducing the viral replication protein(s) in trans. For example, when ori P (the origin of replication on Epstein-Barr virus) is utilized, EBNA-I can be expressed transiently or stably. EBNA-1 will initiate replication from the integrated ori P locus. The replication will extend from the origin bi-directionally. As each replication product is created, it too can initiate replication. As a result, many copies of the viral origin and flanking genomic sequences including the gene of interest are created. This higher copy number allows the cells to produce larger amounts of the gene of interest.

At some frequency, the replication product will recombine to form a circular molecule containing flanking genomic sequences, including the gene of interest. Cells that contain circular molecules with the gene of interest can be isolated by single cell cloning and analysis by Hirt extraction and Southern blotting. Once purified, the cell containing the episomal genomic locus at elevated copy number (typically 10-50 copies) can be propagated in culture. To achieve higher amplification, the episome can be further boosted by including a second origin adjacent to the first in the original construct. For example, T antigen can be used to boost the copy number of ori P/SV40 episomes to a copy number of -1000 (Heinzel et al., J. Virol. 62:3738 (1988)). This substantial increase in copy number can dramatically increase protein expression.

The invention encompasses over-expression of endogenous genes both *in vivo* and *in vitro.* Therefore, the cells could be used *in vitro* to produce desired amounts of a gene product or could be used *in vivo* to provide that gene product in the intact animal.

The invention also encompasses the proteins produced by the methods described herein. The proteins can be produced from either known, or previously unknown genes. Examples of known proteins that can be produced by this method include, but are not limited to, erythropoietin, insulin, growth hormone, glucocerebrosidase, tissue plasminogen activator, granulocyte-colony stimulating factor, granulocyte/macrophage colony stimulating factor, interferon α, interferon β, interferon γ, interleukin-2, interleukin-6, interleukin-11, interleukin-12, TGF β. blood clotting factor V, blood clotting factor VII, blood clotting factor VIII, blood clotting factor IX, blood clotting factor X, TSH-β, bone growth factor 2, bone growth factor-7, tumor necrosis factor, alpha-1 antitrypsin, anti-thrombin III, leukemia inhibitory factor, glucagon, Protein C, protein kinase C, macrophage colony stimulating factor, stem cell factor, follicle stimulating hormone β, urokinase, nerve growth factors, insulin-like growth factors, insulinotropin, parathyroid hormone, lactoferrin, complement inhibitors, platelet derived growth factor, keratinocyte growth factor, neurotropin-3, thrombopoietin, chorionic gonadotropin, thrombomodulin, alpha glucosidase, epidermal growth factor, FGF, macrophage-colony stimulating factor, and cell surface receptors for each of the above-described proteins.

Where the protein product from the activated cell is purified, any method of protein purification known in the art may be employed. ***Isolation of Cells Containing Activated Membrane Protein-Encoding Genes***

Genes that encode membrane associated proteins are particularly interesting from a drug development standpoint. These genes and the proteins they encode can be used, for example, to develop small molecule drugs using combinatorial chemistry libraries and high through-put screening assays. Alternatively, the proteins or soluble forms ofthe proteins (e.g., truncated proteins lacking the transmembrane region) can be used as therapeutically active agents in humans or animals. Identification of membrane proteins can also be used to identify new ligands (e.g., cytokines, growth factors, and other effector molecules) using two hybrid approaches or affinity capture techniques. Many other uses of membrane proteins are also possible.

Current approaches to identifying genes that encode integral membrane proteins involve isolation and sequencing of genes from cDNA libraries. Integral membrane proteins are then identified by ORF analysis using hydrophobicity plots capable of identifying the transmembrane region of the protein. Unfortunately, using this approach a gene encoding an integral membrane protein can not be identified unless the gene is expressed in the cells used to produce the cDNA library. Furthermore, many genes are only expressed in very rare cells, during short developmental windows, and/or at very low levels. As a result, these genes can not be efficiently identified using the currently available approaches.

The present invention allows endogenous genes to be activated without any knowledge of the sequence, structure, function, or expression profile of the genes. Using the disclosed methods, genes may be activated at the transcription level only, or at both the transcription and translation levels. As a result, proteins encoded by the activated endogenous gene can be produced in cells containing the integrated vector. Furthermore, using specific vectors disclosed herein, the protein produced from the activated endogenous gene can be modified, for example, to include an epitope tag. Other vectors (*e.g*., vectors 12-17 described above) may encode a signal peptide followed by an epitope tag. This vector can be used to isolate cells that have activated expression of an integral membrane protein (see Example 5 below). This vector can also be used to direct secretion of proteins that are not normally secreted.

Thus, also referred to are methods for identifying an endogenous gene encoding a cellular integral membrane protein or a transmembrane protein. Such methods may comprise one or more steps. For example, one such method may comprise (a) introducing one or more vectors of the invention into a cell; (b) allowing the vector to integrate into the genome of the cell by non-homologous recombination; (c) allowing over-expression of an endogenous gene in the cell by upregulation of the gene by the transcriptional regulatory sequence contained on the integrated vector construct; (d) screening the cell for over-expression of the endogenous gene; and (e) characterizing the activated gene to determine its identity as a gene encoding a cellular integral membrane protein. In related embodiments, such methods further comprising isolating the activated gene from the cell prior to characterizing the activated gene.

To identify genes that encode integral membrane proteins, vectors integrated into the genome of cells will comprise a regulatory sequence linked to an exonic sequence containing a start codon, a signal sequence, and an epitope tag, followed by an unpaired splice donor site. Upon integration and activation of an endogenous gene, a chimeric protein is produced containing the signal peptide and epitope tag from the vector fused to the protein encoded by the downstream exons of the endogenous gene. This chimeric protein, by virtue of the presence of the vector encoded signal peptide, is directed to the secretory pathway where translation ofthe protein is completed and the protein is secreted. If, however, the activated endogenous gene encodes an integral membrane protein, and the transmembrane region of that gene is encoded by exons located 3' of the vector integration site, then the chimeric protein will go to the cell surface, and the epitope tag will be displayed on the cell surface. Using known methods of cell isolation (for example flow cytometric sorting, magnetic bead cell sorting, immunoadsorption, or other methods that will be familiar to one of ordinary skill in the art), antibodies to the epitope tag can then be used to isolate the cells from the population that display the epitope tag and have activated an integral membrane encoding gene. These cells can then be used to study the function of the membrane protein. Alternatively, the activated gene may then be isolated from these cells using any art-known method, *e.g*., through hybridization with a DNA probe specific to the vector-encoded exon to screen a cDNA library produced from these cells, or using the genetic constructs described herein.

The epitope tag encoded by the vector exon may be a short peptide capable of binding to an antibody, a short peptide capable of binding to a substance (e.g., poly histidine/ divalent metal ion supports, maltose binding protein/maltose supports, glutathione S-transferase/glutathione support), or an extracellular domain (lacking a transmembrane domain) from an integral membrane protein for which an antibody or ligand exists. It will be understood, however, that other types of epitope tags that are familiar to one of ordinary skill in the art may be used equivalently in accordance with the invention.

Having now described the present invention in detail, the same will be more clearly understood by reference to the following examples, which are included herewith for purposes of illustration only and are not intended to be limiting of the invention.

### EXAMPLES

### Example 1: Transfection of Cells for Activation of Endogenous Gene Expression

### Method: Construction of pRIG-1

Human DHFR was amplified by PCR from cDNA produced from HT 1080 cells by PCR using the primers DHFR-F1

(5' TCCTTCGAAGCTTGTCATGGTTGGTTCGCTAAACTGCAT 3') (SEQ ID NO:1) and DHFR-R1 (5' AAACTTAAGATCGATTAATCATTC-TTCTCATATACTTCAA 3') (SEQ ID NO:2), and cloned into the T site in pTARGET™ (Promega) to create pTARGET:DHFR. The RSV promoter was isolated from PREP9 by digestion with *NheI* and *XbaI* and inserted into the *NheI* site of pTARGET:DHFR to create pTgT:RSV+DHFR. Oligonucleotides JH169 (5' ATCCACCATGGCTACAGGTGAGTACTCG 3') (SEQ ID NO:3) and JH170 (5' GATCCGAGTACTCACCTGTAGCCATGGTGGATTTAA 3') (SEQ ID NO:4) were annealed and inserted into the I-Ppo-I and *NheI* sites of pTgT:RSV+DHFR to create pTgT:RSV+DHFR+Exl. A 279 bp region corresponding to nucleotides 230-508 of pBR322 was PCR amplified using primers Tet F1 (5' GGCGAGATCTAGCGCTATATGCGTTGATGCAAT 3') (SEQ ID NO:5)and Tet F2 (5' GGCCAGATCTGCTACCTTAAGAGAGCCG-AAACAAGCGCTCATGAGCCCGAA 3') (SEQ ID NO:6). Amplification products were digested with *BglII* and cloned into the *BamHI* site of pTgT:RSV+RSV+DHFR+Exl to create pRIG-l.

### Transfection -- Creation of pRIG-1 Gene Activation Library in HT1080 Cells

To activate gene expression, a suitable activation construct is selected from the group of constructs described above. The selected activation construct is then introduced into cells by any transfection method known in the art. Examples of transfection methods include electroporation, lipofection, calcium phosphate precipitation, DEAE dextran, and receptor mediated endocytosis. Following introduction into the cells, the DNA is allowed to integrate into the host cell's genome via non-homologous recombination. Integration can occur at spontaneous chromosome breaks or at artificially induced chromosomal breaks.

***Method**:* Transfection of human cells with pRIG-1. 2x10⁹ HH 1 cells, an HPRT⁻ subclone of HT1080 cells, was grown in 150 mm tissue culture plates to 90% confluency. Media was removed from the cells and saved as conditioned media (see below). Cells were removed from the plate by brief incubation with trypsin, added to media/10% fetal bovine serum to neutralize the trypsin, and pelleted at 1000 rpm in a Jouan centrifuge for 5 minutes. Cells were washed in 1 X PBS, counted, and repelleted as above. The cell pellet was resuspended at 2.5 x 10⁷ cells/ml final in 1X PBS (Gibco BRL Cat #14200-075). Cells were then exposed to 50 rads of γ irradiation from a ¹³⁷Cs source. pRIG1 was linearized with *Bam*HI*,* purified with phenol/chloroform, precipitated with ethanol, and resuspended in PBS. Purified and linearized activation construct was added to the cell suspension to produce a final concentration of 40 µg/ml. The DNA/irradiated cell mixture was then mixed and 400 µl was placed into each 0.4 cm electroporation cuvettes (Biorad). The cuvettes were pulsed at 250 Volts, 600 µFarads, 50 Ohms using an electroporation apparatus (Biorad). Following the electric pulse, the cells were incubated at room temperature for 10 minutes, and then placed into αMEM/10%FBS containing penicillin/streptomycin (GibcoBRL). The cells were then plated at approximately 7x10⁶ cells/150 mm plate containing 35 ml αMEM/10% FBS/penstrep (33% conditioned media/67% fresh media). Following a 24 hour incubation at 37°C, G418 (Gibco/BRL) was added to each plate to a final concentration of 500 µg/ml from a 60 mg/ml stock. After 4 days of selection, the media was replaced with fresh αMEM/10% FBS/penstrep/500 µg/ml G418. The cells were then incubated for another 7-10 days and the culture supernatant assayed for the presence of new protein factors or stored at -80 °C for later analysis. The drug resistant clones can be stored in liquid nitrogen for later analysis.

### Example 2: Use of Ionizing Irradiation to Increase the Frequency and Randomness of DNA Integration

***Method**.* HH1 cells were harvested at 90% confluency, washed in 1x PBS, and resuspended at a cell concentration of 7.5 x 10⁶ cells/ml in 1X PBS. 15 µg linearized DNA (pRIG-1) was added to the cells and mixed. 400 µl was added to each electroporation cuvette and pulsed at 250 Volts, 600 µFarads, 50 Ohms using an electroporation apparatus (Biorad). Following the electric pulse, the cells were incubated at room temperature for 10 minutes, and then placed into 2.5 ml αMEM/10%FBS/1X penstrep. 300 µl of cells from each shock were irradiated at 0, 50, 500, and 5000 rads immediately prior to or at either 1 hour or 4 hours post transfection. Immediately following irradiation, the cells were plated onto tissue culture plates in complete medium. At 24 hours post plating, G418 was added to the culture to a final concentration of 500 µg/ml. At 7 days post-selection, the culture medium was replaced with fresh complete medium containing 500 µg/ml G418. At 10 days post selection, medium was removed from the plate, the colonies were stained with Coomassie Blue/90% methanol/10% acetic acid and colonies with greater than 50 cells were counted.

### Example 3: Use of Restriction Enzymes to Generate Random, Semi-random, or Targeted Breaks in the Genome

***Method**:* HHI cells were harvested at 90% confluence, washed in 1x PBS, and resuspended at a cell concentration of 7.5 x 10⁶ cells/ml in I X PBS. To test the efficiency of integration, 15 µg linearized DNA (PGK-βgeo) was added to each 400 µl aliquot of cells and mixed. To several aliquots of cells, restriction enzymes *Xba*I*. Not*I*, Hind*III*, Ippo*I (10-500 units) were then added to separate cell/DNA mixture. 400 µl was added to each electroporation cuvette and pulsed at 250 Volts, 600 µFarads, 50 Ohms using an electroporation apparatus (Biorad). Following the electric pulse, the cells were incubated at room temperature for 10 minutes, and then placed into 2.5 ml αMEM/10%FBS/1X penstrep. 300 µl of 2.5 ml total cells from each shock were plated onto tissue culture plates in complete media. At 24 hours post plating, G418 was added to the culture to a final concentration of 600 mg/ml. At 7 days post-selection, the media was replaced with fresh complete media containing 600 µg/ml G418. At 10 days post selection, media was removed from the plate, the colonies were stained with Coomassie Blue/90% methanol/10% acetic acid and colonies with greater than 50 cells were counted.

### Example 4: Amplification by Selecting for Two Amplifiable Markers Located on the Integrated Vector

Following integration of the vector into the genome of a host cell, the genetic locus may be amplified in copy number by simultaneous or sequential selection for one or more amplifiable markers located on the integrated vector. For example, a vector comprising two amplifiable markers may be integrated into the genome, and expression of a given gene (*i.e*., a gene located at the site of vector integration) can be increased by selecting for both amplifiable markers located on the vector. This approach greatly facilitates the isolation of clones of cells that have amplified the correct locus (*i.e*., the locus containing the integrated vector).

Once the vector has been integrated into the genome by nonhomologous recombination, individual clones of cells containing the vector integrated in a unique location may be isolated from other cells containing the vector integrated at other locations in the genome. Alternatively, mixed populations of cells may be selected for amplification.

Cells containing the integrated vector are then cultured in the presence of a first selective agent that is specific for the first amplifiable marker. This agent selects for cells that have amplified the amplifiable marker either on the vector or on the endogenous chromosome. These cells are then selected for amplification of the second selectable marker by culturing the cells in the presence of a second selective agent that is specific for the second amplifiable marker. Cells that amplified the vector and flanking genomic DNA will survive this second selective step, whereas cells that amplified the endogenous first amplifiable marker or that developed non-specific resistance will not survive. Additional selections may be performed in similar fashion when vectors containing more than two (*e.g*., three, four, five, or more) amplifiable markers are integrated into the cell genome, by sequential culturing of the cells in the presence of selective agents that are specific for the additional amplifiable markers contained on the integrated vector. Following selection, surviving cells are assayed for level of expression ofa desired gene, and the cells expressing the highest levels are chosen for further amplification. Alternatively, pools of cells resistant to both (if two amplifiable markers are used) or all (if more than two amplifiable markers are used) of the selective agents may be further cultured without isolation of individual clones. These cells are then expanded and cultured in the presence of higher concentrations of the first selective agent (usually twofold higher). The process is repeated until the desired expression level is obtained.

Alternatively, cells containing the integrated vector may be selected simultaneously for both (if two are used) or all (if more than two are used) of the amplifiable markers. Simultaneous selection is accomplished by incorporating both selection agents (if two markers are used) or all of the selection agents (if more than two markers are used) into the selection medium in which the transfected cells are cultured. The majority of surviving cells will have amplified the integrated vector. These clones can then be screened individually to identify the cells with the highest expression level, or they can be carried as a pool. A higher concentration of each selective agent (usually twofold higher) is then applied to the cells. Surviving cells are then assayed for expression levels. This process is repeated until the desired expression levels are obtained.

By either selection strategy (i. e., simultaneous or sequential selection), the initial concentration of selective agent is determined independently by titrating the agent from low concentrations with no cytotoxicity to high concentrations that result in cell death in the majority of cells. In general, a concentration that gives rise to discrete colonies (*e.g*., several hundred colonies per 100,000 cells plated) is chosen as the initial concentration.

### Example 5: Isolation of cDNAs Encoding Transmembrane Proteins

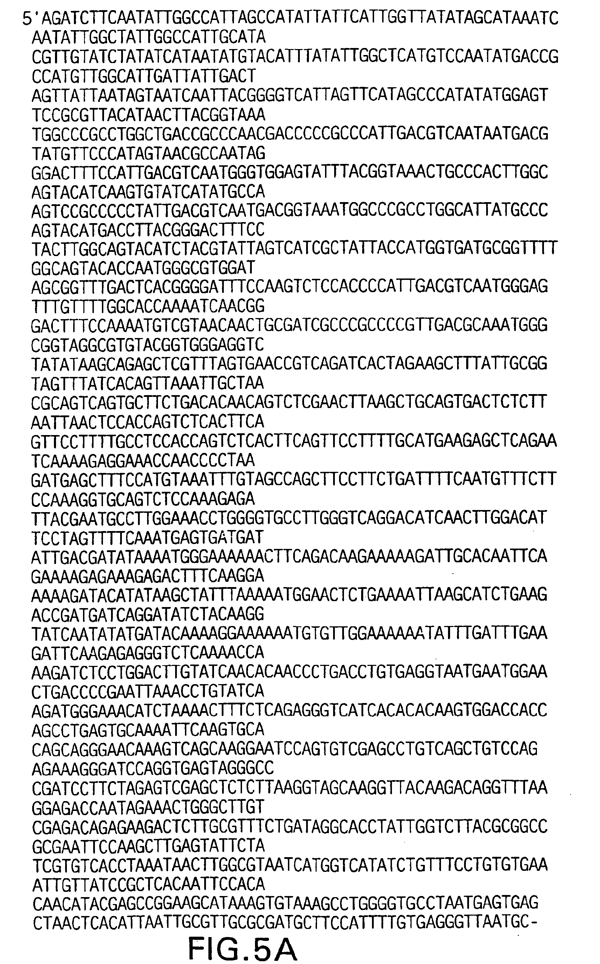

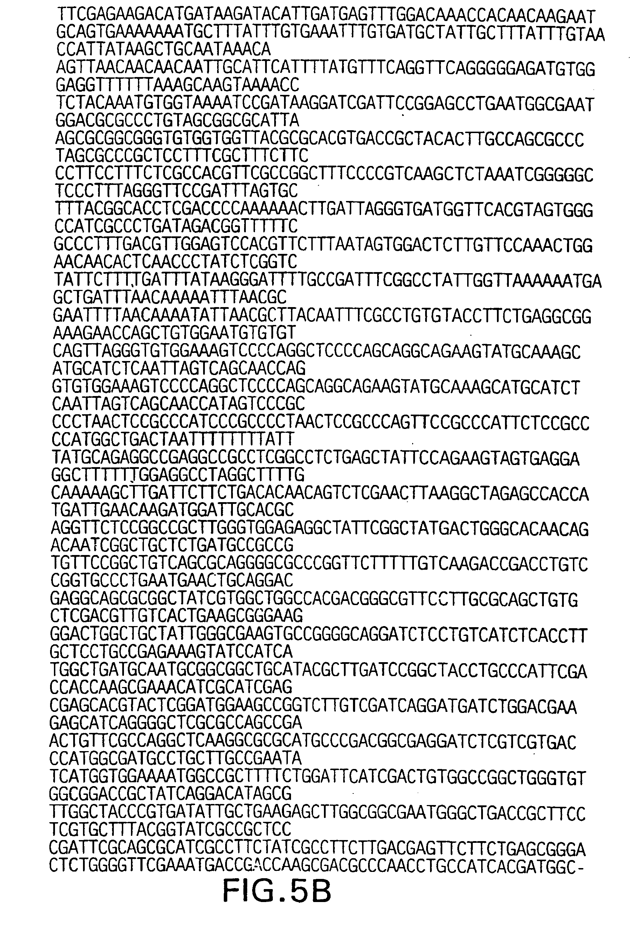

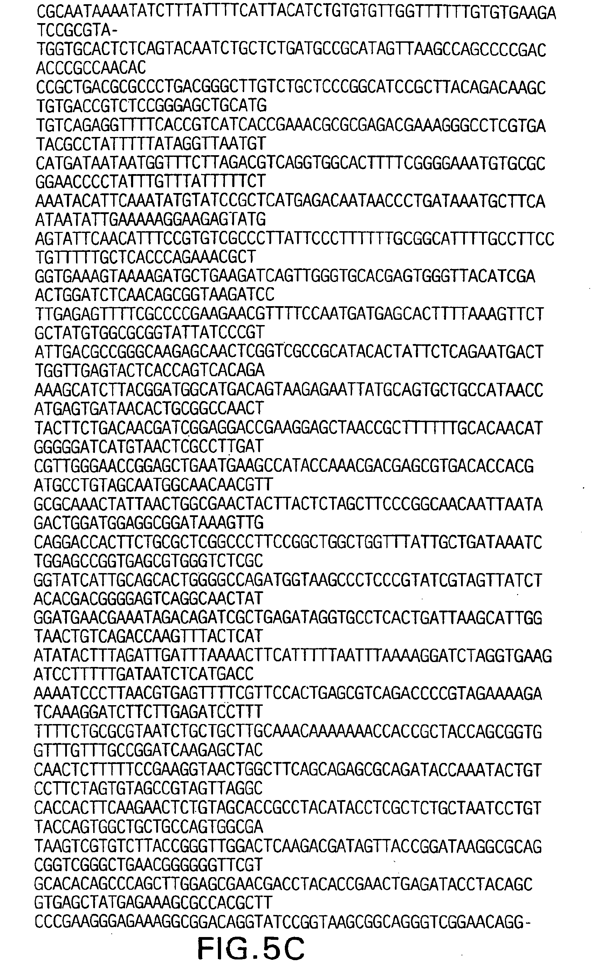

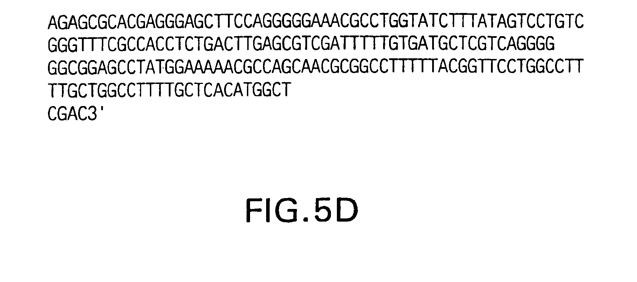

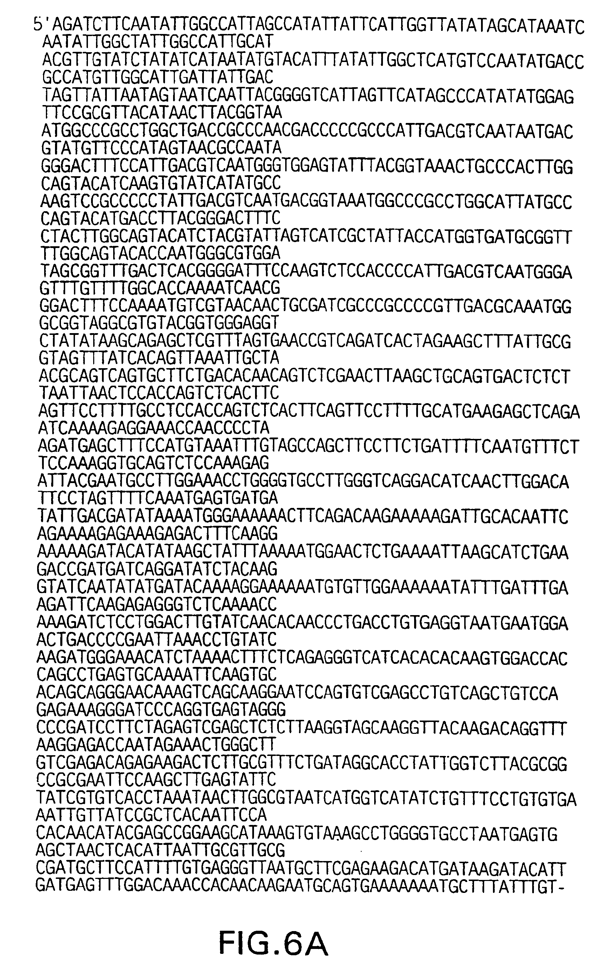

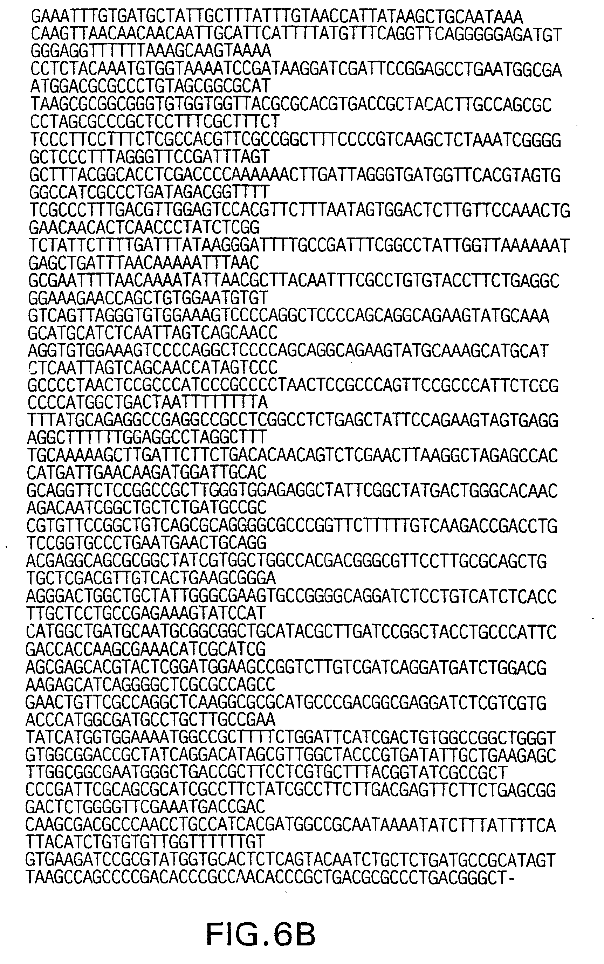

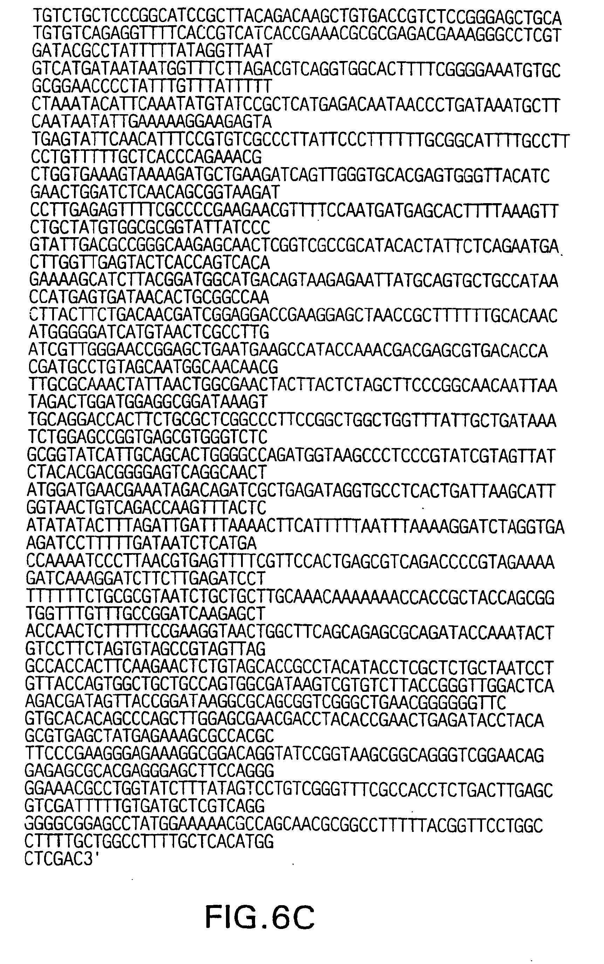

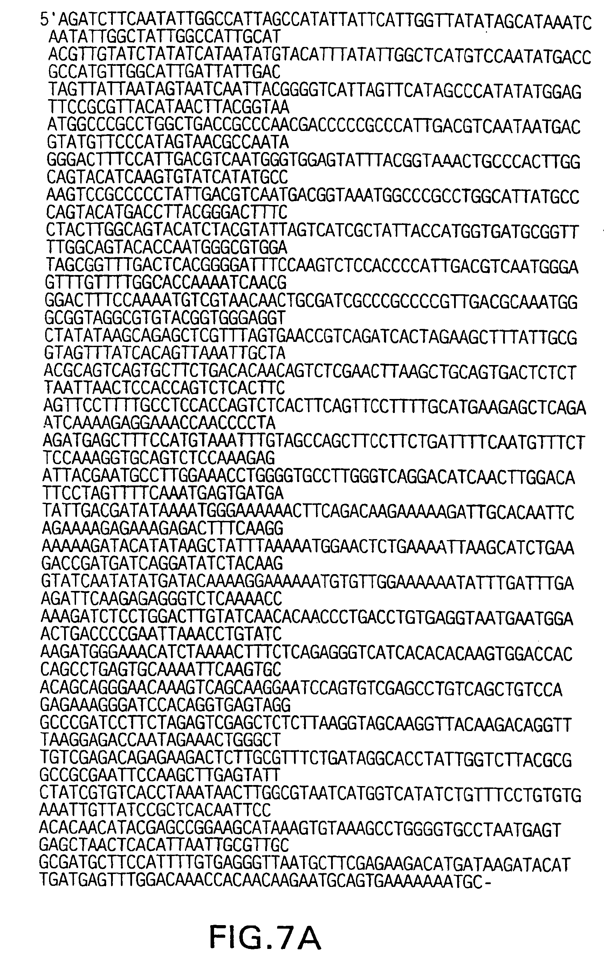

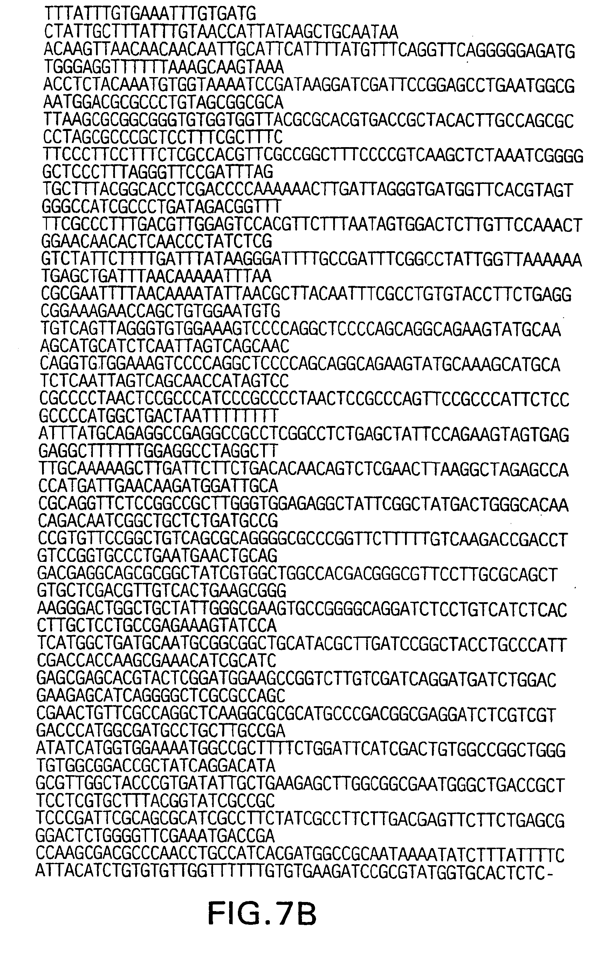

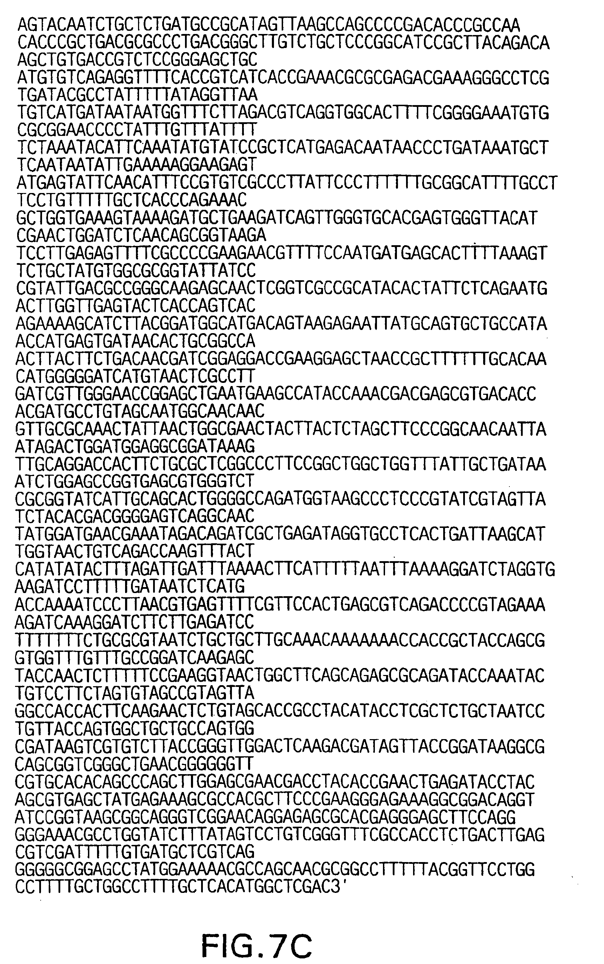

pRIG8R1-CD2 (Fig. 5A-5D; SEQ ID NO:7), pRIG8R2-CD2 (Fig. 6A-6C; SEQ ID NO:8), and pRIG8R3-CD2 (Fig. 7A-7C; SEQ ID NO:9) vectors contain the CMV immediate early gene promoter operably linked to an exon followed by an unpaired splice donor site. The exon on the vector encodes a signal peptide linked to the extra-cellular domain of CD2 (lacking an in frame stop codon). Each vector encodes CD2 in a different reading frame relative to the splice donor site.

To create a library of activated genes, 2 x 10⁷ cells were irradiated with 50 rads from a ¹³⁷Cs source and electroporated with 15 µg of linearized pRIG8R1-CD2 (SEQ ID NO:7). Separately, this was repeated with pRIG8R2-CD2 (SEQ ID NO:8), and again with pRIG8R3-CD2 (SEQ ID NO:9). Following transfection, the three groups of cells were combined and plated into 150 mm dishes at 5 x 10⁶ transfected cells per dish to create library #1. At 24 hours post transfection, library #1 was placed under 500 µg/ml G418 selection for 14 days. Drug resistant clones containing the vector integrated into the host cell genome were combined, aliquoted, and frozen for analysis. Library #2 was created as described above, except that 3 x 10⁷ cells, 3 x 10⁷ cells and 1 x 10⁷ cells were transfected with pPIG8R1-CD2, pRIG8R2-CD2, and pRIG8R3-CD2, respectively.

To isolate cells containing activated genes encoding integral membrane proteins, 3 x 10⁶ cells from each library were cultured and treated as follows:
Cells were trypsinized using 4 mls of Trypsin- EDTA.
After the cells had released, the trypsin was neutralized by addition of 8 ml of alpha MEM/10% FBS.
The cells were washed once with sterile PBS and collected by centrifugation at 800 x g for 7 minutes.
The cell pellet was resuspended in 2ml of alpha MEM/10% FBS. 1 ml was used for sorting while the other 1 ml was replated in alpha MEM/10% FBS containing 500 µg/ml G-418, expanded and saved.
The cells used for sorting were washed once with sterile alpha MEM/10% FBS and collected by centrifugation at 800 x g for 7 minutes.
The supernatant was removed and the pellet resuspended in 1 ml of alpha MEM/10% FBS. 100 µl of these cells was removed for staining with the isotype control.
200 µl of Anti-CD2 FITC (Pharmingen catalog # 30054X) was added to the 900 µl of cells while 20 µl of the Mouse IgG₁ isotype control (Pharmingen catalog # 33814X) was added to the 100 µl of cells. The cells were incubated, on ice, for 20 minutes.
To the tube that contained the cells stained with the Anti-Human CD2 FITC, 5 ml of PBS/1% FBS were added. To the isotope control, 900 µl of PBS/1% FBS were added. The cells were collected by centrifugation at 600 x g for 6 minutes.
The supernatant from the tubes was removed. The cells that had been stained with the isotype control were resuspended in 500 µl of alpha MEM/10% FBS, and the cells that had been stained with anti-CD2- FITC were resuspended in 1,5 ml alpha MEM/10% FBS.

Cells were sorted through five sequential sorts on a FACS Vantage Flow Cytometer (Becton Dickinson Immunocytometry Systems; Mountain View, CA). In each sort, the indicated percentage oftotal cells, representing the most strongly fluorescent cells (see below) were collected, expanded, and resorted. HT1080 cells were sorted as a negative control. The following populations were sorted and collected in each sort:

| | **Library #1** | **Library #2** | **Library #3** |
|---|---|---|---|
| **Sort #1** | 500,000 cells collected (top 10%) | 100,000 cells collected (top 10%) | 40,000 cells collected (top 10%) |
| **Sort #2** | 300,000 cells collected (top 5%) | 220,000 cells collected (top 11%) | 14,000 cells collected (top 5%) |
| **Sort #3** | 90,000 cells collected (top 5%) | 40,000 cells collected (top 10%) | 120,000 cells collected (top 10%) |
| **Sort #4** | 600,000 cells collected (top 40%) | (**a**) 6,000 cells collected (top 5%); (**b**) 10,000 cells collected (next 5%) | 280,000 cells collected (top 13%) |
| **Sort #5** | (**a**) 260,000 cells collected (top 10%); (**b**) 530,000 cells collected (next 25%) | (**a**) from group (a) of sort #4, 100,000 cells collected (top 10%), and 350,000 cells collected (next 35%); (**b**) from group (b) of sort #4, 120,000 cells collected (top 10%) | (Not done) |

Cells from each of the final sorts for each library were expanded and stored in liquid nitrogen.

### Isolation of activated genes from FACS-sorted cells

Once cells had been sorted as described above, activated endogenous genes from the sorted cells were isolated by PCR-based cloning One of ordinary skill will appreciate, however, that any art-known method of cloning of genes may be equivalently used to isolate activated genes from FACS-sorted cells.

Genes were isolated by the following protocol:
(1) Using PolyATract System 1000 mRNA isolation kit (Promega), mRNA was isolated from 3x10⁷ CD2+ cells (sorted 5 rounds by FACS, as described above) from libraries #1 and #2.
(2) After mRNA isolation, the concentration of mRNA was determined by diluting 0.5 µl of isolated mRNA into 99.5 µl water and measuring OD²⁶⁰. 21 µg of mRNA were recovered from the CD2+ cells.
(3) First strand cDNA synthesis was then carried out as follows:
(a) While the PCR machine was holding at 4°C, first strand reaction mixtures were set up by sequential addition of the following components:
   41 µl DEPC-treated ddH₂O
   4 µl 10mM each dNTP
   8 µl 0.1 MDTT
   16 µl 5x MMLV first strand buffer (Gibco-BRL)
   5 µl (10pmol/µl) ofthe consensus poly adenylation site primer GD.R1 (SEQ ID NO:10)*
   1 µl RNAsin (Promega) 3 µl (1.25 µg/µl) mRNA.
   *Note: GD.R1, 5'TTTTTTTTTTTTCGTCAGCGGCCGCATCNNNNTTT-ATT 3' (SEQ ID NO:10), is a "Gene Discovery" primer for first strand cDNA synthesis of mRNA; this primer is designed to anneal to the poly-adenylation signal AATAAA and downstream poly-A region. This primer will introduce a *Not*l site into the first strand. Once samples had been made up, they were incubated as follows:
(b) 70° for 1 min.
(c) 42° hold.
   2 µl of 400 U/µl SuperScript II (Gibco-BRL; Rockville, MD) was then added to each sample, to give a final total volume of 82 µl.
   After approximately three minutes, samples were incubated as follows:
(d) 37° for 30 min.
(e) 94° for 2 min.
(f) 4° for 5 min.
   2 µl of 20 U/µl RNace-IT (Stratagene) was then added to each sample, and samples were incubated at 37° for 10 min.

(4) Following first strand synthesis, cDNA was purified using a PCR cleanup kit (Qiagen) as follows:
(a) 80 µl of the first strand reaction were transferred to a 1.7 ml siliconized eppendorf tube and adding 400 µl of PB.
(b) Samples were then transferred to a PCR clean-up column and centrifuged for two minutes at 14,000 RPM.
(c) Columns were then disassembled, flowthrough decanted, 750 of µl PE were added to pellets, and tubes were centrifuged for two minutes at 14,000 RPM.
(d) Columns were disassembled and flowthrough decanted, and tubes then centrifuged for two minutes at 14,000 RPM to dry resin.
(e) cDNA was then eluted using 50 µl of EB through transferring column to a new siliconized eppendorf tube which was then centrifuged for two minutes at 14,000 RPM.

(5) Second strand cDNA synthesis was then carried out as follows:
(a) Second strand reaction mixtures were set up at RT, through the sequential addition of the following components:

| | |
|---|---|
| ddH₂0 | 55 µl |
| 10 x PCR buffer | 10 µl |
| 50 mM MgCl₂ | 5 µl |
| 10 mM dNTPs | 2 µl |
| 25 pmol/µl RIG.751-Bio* | 4 µl |
| 25 pmol/µl GD.R2** | 4 µl |
| First strand product | 20 µl |

| | |
|---|---|
| "Note: RIG.F751-Bio, 5' Biotin-CAGATCACTAGAAGCTTTATTGCGG 3' (SEQ ID NO:11), anneals at the cap-site of the transcript expressed from pRIG vectors. **Note: GD.R2, 5' TTTTCGTCAGCGGCCGCATC 3' (SEQ ID NO:12), is a primer used to PCR amplify cDNAs generated using primer GD.R1 (SEQ ID NO:10). GD.R2 is a sub-sequence of GD.R1 with matching sequence up to the degenerate bases preceding the polyA signal sequence. | |

(b) Start second strand synthesis:
94°C for 1 min;
add 1 µl *Taq* (5U/µl, Gibco-BRL);
add 1 µl Vent DNA pol (0.1U/µl, New England Biolabs).

(c) Incubate at 63 °C for 2 min.
(d) Incubate at 72°C for 3 min.
(e) Repeat step (b) four times.
(f) Incubate at 72°C for 6 min.
(g) Incubate at 4 ° C (hold)
(h) END
(6) 200 µl of 1 mg/ml Streptavidin-Paramagnetic Particles (SA-PMP) were then prepared by washing three times with STE.
(7) The products of the second strand reaction were added directly to the SA-PMPs and incubated at RT for 30 minutes.
(8) After binding, SA-PMPs were collected through the use of the magnet, and flowthrough material recovered.
(9) Beads were washed three times with 500 µl STE.
(10) Beads were resuspended in 50 µl of STE and collected at the bottom of the tube using the magnet. STE supernatant was then carefully pipetted off.
(11) Beads were resuspended in 50 µl of ddH₂0 and placed into a 100°C water bath for two minutes, to release purified cDNA from PMPs.
(12) Purified cDNA was recovered by collecting PMPs on the magnet and carefully removing the supernatant containing the cDNA.
(13) Purified products were transferred to a clean tube and centrifuged at 14,000 RPM for two minutes to remove all of the residual PMPs.
(14) A PCR reaction was then carried out to specifically amplify RIG activated cDNAs, as follows:
(a) PCR reaction mixtures were set up at RT, through the sequential addition of the following components:

| | |
|---|---|
| H₂O | 59 µl |
| 10 x PCR buffer | 10 µl |
| 50 mM MgCl₂ | 5 µl |
| 10 mM dNTPs | 2 µl |
| 25pmol/µl RIG.F781* | 2 µl |
| 25 pmol/µl GD.R2 | 2 µl |
| second strand product | 20 µl |

| | |
|---|---|
| *Note: RIG.F781, 5' ACTCATAGGCCATAGAGGCCTATCACAG-TTAAATTGCTAACGCAG 3' (SEQ ID NO:13), anneasl downstream of GD.F1 GD.F3, GD.F5-Bio, and RIG.F751-Bio, and adds an *Sfi*I site for 5' cloning of cDNAs. This primer is used in nested PCR amplification of RIG Exon 1 specific second strand cDNAs. | |

(b) Start thermal cycler:
94°C for 3 min;
add 1 µl of *Taq* (5U/µl; Gibco-BRL):
add 1 µl of 0.1 U/µl Vent DNA polymerase (New England Biolabs)
   PCR was then carried out by 10 cycles of steps (c) to (e):

(c) 94°C for 30 sec.
(d) 60°C for 40 sec.
(e) 72°C for 3 min.
PCR was then completed by carrying out the following steps:
(f) 94°C for 30 sec.
(g) 60°C for 40 sec.
(h) 72°C for 3 min.
(i) 72°C + 20 sec each cycle for 10 cycles
(j) 72°C for 5 min
(k) 4°C hold.
(15) After elution of library material with 50 µl EB, samples were digested by adding 10 µl of NEB Buffer 2, 40 µl of dH₂O and 2 µl of *Sfi*I and digesting for 1 hour at 50 °C, to cut the 5' end of the cDNA at the *Sfi*I site encoded by the forward primer (RIG.F781; SEQ ID NO:13).
(16) Following *Sfi*I digestion, 5 µl of 1M NaCl and 2 µl *of Not*I were added to each sample, and samples digested for one hour at 37°C, to cut the 3' end of the cDNA at the *Not*I site encoded by the first strand primer (GD.R1; SEQ ID NO:10).
(17) The digested cDNA was then separated on a 1% low melt agarose gel. cDNAs ranging in size from 1.2Kb to 8Kb were excised from the gel.
(18) cDNA was recovered from the excised agarose gel using Qiaex II Gel Extraction (Qiagen). 2 µl of cDNA (approximately 30mg) was ligated to 7µl (35ng) of pBS-HSB (linearized with *Sfi*I/*Not*I) in a total volume of 10 µl of 1X T4 ligase buffer (NEB), using 400 units of T4 DNA ligase (NEB).
(19) 0.5 µl of the ligation reaction mixture from step (18) was transformed into *E. coli* DH10B.
(20) 103 colonies/0.5 µl ligated DNA were recovered.
(21) These colonies were screened for exons using the primers M13F20 and JH182 (RiG Exon1 specific) through PCR in 12.5 µl volumes as follows:
(a) 100 µl of LB (with selective antibiotic) were dispensed into the appropriate number of 96-well plates.
(b) Single colonies were picked and inoculated into individual wells of the 96-well plate, and the plate placed into a 37°C incubator for 2-3 hours without shaking.
(c) A PCR reaction "master mix" was prepared on ice, as follows:

| *# of 96-Well Plates:* | ***1 Plate*** | ***2 Plates*** | ***3 Plates*** | ***4 Plates*** |
|---|---|---|---|---|
| *Total # of 12.5 µl PCR rxns:* | ***96*** | ***192*** | ***288*** | ***384*** |
| *dH₂O* | *755 µl* | *1.47 ml* | *2.20 ml* | *2.94 ml* |
| *5X PCR Premix-4* | *250 µl* | *500 µl* | *750 µl* | *1.0 ml* |
| *F Primers premix (25 pmol*/*µl)* | *10 µl* | *20 µl* | *30 µl* | *40 µl* |
| *R Primers premix (25 pmol*/*µl)* | *10 µl* | *20 µl* | *30 µl* | *40 µl* |
| *RNace-It Cocktail* | *3. 2 µl* | *6.3 µl* | *9.6 µl* | *12.8 µl* |
| *Taq Polymerase (5 U*/*µl)* | *3.2 µl* | *6.3 µl* | *9.6 µl* | *12.8 µl* |
| *Total Volume (ml)* | *1*.*01* | *2. 02* | *3. 03* | *4. 04* |

(d) 10 µl of the master mix were dispensed into each well of the PCR reaction plate.
(e) 2.5 µl from each 100 µl *E. coli* culture were transferred into the corresponding wells of the PCR reaction plate.
(f) PCR was performed, using typical PCR cycle conditions of:
(i) 94°C/2min. (Bacterial lysis and plasmid denaturation)
(ii) 30 cycles of 92°C denaturation for 15 sec; 60°C primer annealing for 20 sec; and 72°C primer extension for 40 sec
(iii) 72°C final extension for 5 min.
(iv) 4 ° C hold.

(g) Bromophenol blue was then added to the PCR reaction; samples were mixed, centrifuged, and then the entire reaction mix was loaded onto an agarose gel.
(23) Of 200 clones screened, 78% were positive for the vector exon. 96 of these clones were grown as minipreps and purified using a Qiagen 96-well turbo-prep following the Qiagen Miniprep Handbook (April 1997).
(24) Many duplicate clones were eliminated though simultaneous digestion of 2 µl of DNA with *Not*I*, Bam*HI, *Xho*I*, Xba*I*, Hind*III, *EcoR*I in NEB Buffer 3, in a total volume of 22 µl, followed by electrophoresis on a 1% agarose gel.

### Results:

Two different cDNA libraries were screened using this protocol. In the first library (TMT#1), eight of the isolated activated genes were sequenced. Of these eight genes, four genes encoded known integral membrane proteins and six were novel genes. In the second library (TMT#2), 11 isolated activated genes were sequenced. Of these 11 genes, one gene encoded a known integral membrane protein, one gene encoded a partially sequenced gene homologous to an integral membrane protein, and nine were novel genes. In all cases where the isolated gene correspond to a characterized known gene, that gene was an integral membrane protein.

Exemplary significant alignments (obtained from GenBank) for genes isolated from each library are shown below:

### TMT#1 Significant Alignments:

179761|gb|M76559|HUMCACNLB Human neuronal DHP-sensitive voltage-dependent, calcium channel alpha-2b subunit mRNA complete CDs.
   Length = 3600
>gi|3183979|emb|Y101831HSMEMD H.sapiens mRNA for MEMD protein
   Length = 4235

### TMT#2 Significant Alignments:

>gi|476590|gb|U06715|HSU06715 Human cytochrome B561, HCYTO B561, mRNA, partial CDs.
   Length = 2463
>gi|2189893|gb|AA959959|AA959959 zx66c01.s1 Soares total fetus Nb2HF8 9w Homo sapiens cDNA clone 796414 3' similar to gb:J03171 INTERFERON-ALPHA RECEPTOR PRECURSOR (HUMAN); Length = 431

Having now fully described the present invention in some detail by way of illustration and example for purposes of clarity of understanding, it will be obvious to one of ordinary skill in the art that the same can be performed by modifying or changing the invention within a wide and equivalent range of conditions, formulations and other parameters without affecting the scope of the invention or any specific embodiment thereof, and that such modifications or changes are intended to be encompassed within the scope of the appended claims.

All publications, patents and patent applications mentioned in this specification are indicative of the level of skill of those skilled in the art to which this invention pertains.

## Claims

1. A method for providing a cell that produces a protein product of an endogenous gene, the method comprising:
(a) introducing a genetically-engineered vector construct comprising a transcriptional regulatory sequence for over-expression operably linked to an unpaired splice donor sequence into a eukaryotic cell *in vitro;*
(b) integrating the vector construct into the genome of said eukaryotic cell by non-homologous recombination whereby said transcriptional regulatory sequence and unpaired splice donor sequence become operably linked to the endogenous gene so said splice donor sequence becomes paired with a splice acceptor sequence of the endogenous gene; and
(c) over-expressing, or activating expression of protein encoded by an endogenous gene in said eukaryotic cell by upregulation of said gene by said transcriptional regulatory sequence.

2. The method of claim 1 wherein the method further comprises:
(d) isolating and cloning the over-expressing non-homologously recombinant cell.

3. The method of claim 1 or 2 further comprising isolating the expression product.

4. The method of any one of the preceding claims wherein the vector construct further comprises one or more amplifiable markers.

5. The method of any one of claims 1 to 4 where in the vector construct the transcriptional regulatory sequence is operably linked to a translational start codon and a secretion signal sequence that precede the unpaired splice donor site.

6. The method of any one of claims 1 to 4 where in the vector construct the transcriptional regulatory sequence is operably linked to a translational start codon and an epitope tag that precede the unpaired splice donor site.

7. The method of any one claims 1 to 4 where in the vector construct the transcriptional regulatory sequence is operably linked to a translational start codon, a secretion signal sequence and an epitope tag, that precede the unpaired splice donor site.

8. The method of any one of claims 1 to 4 where in the vector construct the transcriptional regulatory sequence is operably linked to a translational start codon, a secretion signal sequence, an epitope tag and, a sequence-specific protease site all preceding the unpaired splice donor site.

9. The method of any one of the preceding claims, wherein the construct further comprises an internal ribosome entry site for producing a polycistronic message.

10. The method of any one of the preceding claims wherein the construct further comprises two or more amplifiable markers.

11. The method of any one of the preceding claims wherein said transcriptional regulatory sequence is a promoter.

12. The method of claim 11, wherein said promoter is a viral promoter.

13. The method of claim 12, wherein said viral promoter is a cytomegalovirus immediate early promoter.

14. The method of claim 11, wherein the promoter is a nonviral promoter

15. The method of claim 11, wherein said promoter is an inducible promoter.

16. The method of anyone of the preceding claims, wherein the transcriptional regulatory sequence is operably linked to a secretion signal sequence preceding the splice donor site.

17. The method of any one of the preceding claims wherein the transcriptional regulatory sequence is non-retroviral.

18. The method of any one of the preceding claims further comprising introducing double strand breaks into the genomic DNA of said cell prior to or simultaneously with integration of said vector construct.

19. The method of any one of the preceding claims wherein the vector construct is linear.

20. The method of any one of the preceding claims further comprising isolating and cloning the cell producing the protein.

## Patentansprüche

1. Verfahren zum Bereitstellen einer Zelle, die ein Proteinprodukt eines endogenen Gens produziert, wobei das Verfahren folgendes umfaßt:
(a) Einführen eines gentechnisch konstruierten Vektorkonstrukts umfassend eine Transkriptionsregulationssequenz für Überexpression in operativer Verknüpfung mit einer ungepaarten Spleißdonorsequenz in eine eukaroyntische Zelle *in vitro;*
(b) Integrieren des Vektorkonstrukts in das Genom dieser eukaryontischen Zelle durch nichthomologe Rekombination, wodurch die Transkriptionsregulationssequenz und die ungepaarte Spleißdonorsequenz operativ mit dem endogenen Gen so verknüpft werden, daß die Spleißdonorsequenz mit einer Spleißakzeptorsequenz des endogenen Gens gepaart wird; und
(c) Überexprimieren bzw. Aktivieren der Expression eines Proteins, das von einem endogenen Gen codiert wird, in der eukaryontischen Zelle durch Hinaufregulieren des Gens durch die Transkriptionsregulationssequenz.

2. Verfahren nach Anspruch 1, das weiterhin folgendes umfaßt:
(d) Isolieren und Klonieren der überexprimierenden nichthomolog rekombinanten Zelle.

3. Verfahren nach Anspruch 1 oder 2, das weiterhin das Isolieren des Expressionsprodukts umfaßt.

4. Verfahren nach einem der vorhergehenden Ansprüche, wobei das Vektorkonstrukt weiterhin einen oder mehrere amplifzierbare Marker umfaßt.

5. Verfahren nach einem der Ansprüche 1 bis 4, wobei die Transkriptionsregulationssequenz in dem Vektorkonstrukt mit einem Translationsstartcodon und einer Sekretionssignalsequenz, die vor der ungepaarten Spleißdonorstelle liegen, operativ verknüpft ist.

6. Verfahren nach einem der Ansprüche 1 bis 4, wobei die Transkriptionsregulationssequenz in dem Vektorkonstrukt mit einem Translationsstartcodon und einem Epitop-Tag, die vor der ungepaarten Spleißdonorstelle liegen, operativ verknüpft ist.

7. Verfahren nach einem der Ansprüche 1 bis 4, wobei die Transkriptionsregulationssequenz in dem Vektorkonstrukt mit einem Translationsstartcodon, einer Sekretionssignalsequenz und einem Epitop-TAG, die vor der ungepaarten Spleißdonorstelle liegen, operativ verknüpft ist.

8. Verfahren nach einem der Ansprüche 1 bis 4, wobei die Transkriptionsregulationssequenz in dem Vektorkonstrukt mit einem Translationsstartcodon, einer Sekretionssignalsequenz, einem Epitop-TAG und einer sequenzspezifischen Proteasestelle, die alle vor der ungepaarten Spleißdonorstelle liegen, operativ verknüpft ist.

9. Verfahren nach einem der vorhergehenden Ansprüche, wobei das Konstrukt weiterhin eine interne ribosomale Eintrittsstelle zur Erzeugung einer polycistronischen Information umfaßt.

10. Verfahren nach einem der vorhergehenden Ansprüche, wobei das Konstrukt weiterhin zwei oder mehr amplifizierbare Marker umfaßt.

11. Verfahren nach einem der vorhergehenden Ansprüche, wobei es sich bei der Transkriptionsregulationssequenz um einen Promoter handelt.

12. Verfahren nach Anspruch 11, wobei es sich bei dem Promoter um einen Viruspromoter handelt.

13. Verfahren nach Anspruch 12, wobei es sich bei dem Viruspromoter um einen "immediate-early"-Promoter des Cytomegalievirus handelt.

14. Verfahren nach Anspruch 11, wobei es sich bei dem Promoter um einen Promoter, der nicht von einem Virus stammt, handelt.

15. Verfahren nach Anspruch 11, wobei es sich bei dem Promoter um einen induzierbaren Promoter handelt.

16. Verfahren nach einem der vorhergehenden Ansprüche, wobei die Transkriptionsregulationssequenz operativ mit einer Sekretionssignalsequenz, die vor der Spleißdonorstelle liegt, verknüpft ist.

17. Verfahren nach einem der vorhergehenden Ansprüche, wobei die Transkriptionsregulationssequenz nicht von einem Retrovirus stammt.

18. Verfahren nach einem der vorhergehenden Ansprüche, das weiterhin das Induzieren von Doppelstrangbrüchen in der genomischen DNA der Zelle vor oder zugleich mit der Integration des Vektorkonstrukts umfaßt.

19. Verfahren nach einem der vorhergehenden Ansprüche, wobei das Vektorkonstrukt linear ist.

20. Verfahren nach einem der vorhergehenden Ansprüche, das weiterhin das Isolieren und Klonieren der Zelle, die das Protein produziert, umfaßt.

## Revendications

1. Procédé pour mettre à disposition une cellule qui produise un produit protéique d'un gène endogène, le procédé comprenant :
(a) l'introduction d'une construction de vecteur génétiquement modifiée, comprenant une séquence régulatrice de transcription pour une surexpression liée d'une manière opérationnelle à une séquence donneuse d'épissage non appariée dans une cellule eucaryote in vitro ;
(b) l'intégration de la construction de vecteur dans le génome de ladite cellule eucaryote par recombinaison non homologue, ladite séquence régulatrice de transcription et ladite séquence donneuse d'épissage non appariée devenant liée d'une manière opérationnelle au gène endogène de telle sorte que ladite séquence donneuse d'épissage devienne appariée avec la séquence acceptrice d'épissage du gène endogène ; et
(c) la surexpression ou l'activation de l'expression d'une protéine codée par un gène endogène dans ladite cellule eucaryote par régulation à la hausse dudit gène par ladite séquence régulatrice de transcription.

2. Procédé selon la revendication 1, le procédé comprenant en outre :
(d) l'isolement et le clonage de la cellule à recombinaison non homologue en surexpression.

3. Procédé selon la revendication 1 ou 2, qui comprend en outre l'isolement du produit de l'expression.

4. Procédé selon l'une quelconque des revendications précédentes, dans lequel la construction de vecteur comprend en outre un ou plusieurs marqueurs amplifiables.

5. Procédé selon l'une quelconque des revendications 1 à 4, dans lequel, dans la construction de vecteur, la séquence régulatrice de transcription est liée d'une manière opérationnelle à un codon d'initiation de traduction et une séquence signal de sécrétion qui précède le site donneur d'épissage non apparié.

6. Procédé selon l'une quelconque des revendications 1 à 4, dans lequel, dans la construction de vecteur, la séquence régulatrice de transcription est liée d'une manière opérationnelle à un codon d'initiation de traduction et à une étiquette d'épitope qui précède le site donneur d'épissage non apparié.

7. Procédé selon l'une quelconque des revendication 1 à 4, dans lequel, dans la construction de vecteur, la séquence régulatrice de transcription est liée d'une manière opérationnelle à un codon d'initiation de traduction, à une séquence signal de sécrétion et à une étiquette d'épitope, qui précèdent le site donneur d'épissage non apparié.

8. Procédé selon l'une quelconque des revendications 1 à 4, dans lequel, dans la construction de vecteur, la séquence régulatrice de transcription est liée d'une manière opérationnelle à un codon d'initiation de traduction, à une séquence signal de sécrétion, à une étiquette d'épitope et à un site de protéase spécifique de séquence, qui tous précèdent le site donneur d'épissage non apparié.

9. Procédé selon l'une quelconque des revendications précédentes, dans lequel la construction comprend en outre un site d'entrée interne dans le ribosome pour produire un message polycistronique.

10. Procédé selon l'une quelconque des revendications précédentes, dans lequel la construction comprend en outre deux marqueurs amplifiables, ou plus.

11. Procédé selon l'une quelconque des revendications précédentes, dans lequel ladite séquence régulatrice de transcription est un promoteur.

12. Procédé selon la revendication 11, dans lequel ledit promoteur est un promoteur viral.

13. Procédé selon la revendication 12, dans lequel ledit promoteur viral est un promoteur précoce immédiat du cytomégalovirus.

14. Procédé selon la revendication 11, dans lequel le promoteur est un promoteur non viral.

15. Procédé selon la revendication 11, dans lequel ledit promoteur est un promoteur inductible.

16. Procédé selon l'une quelconque des revendications précédentes, dans lequel la séquence régulatrice de transcription est liée d'une manière opérationnelle à une séquence signal de sécrétion précédent le site donneur d'épissage.

17. Procédé selon l'une quelconque des revendications précédentes, dans lequel la séquence régulatrice de transcription est non rétrovirale.

18. Procédé selon l'une quelconque des revendications précédentes, qui comprend en outre l'introduction de cassures double brin dans l'ADN génomique de ladite cellule antérieurement ou simultanément à l'intégration de ladite construction de vecteur.

19. Procédé selon l'une quelconque des revendications précédentes, dans lequel la construction de vecteur est linéaire.

20. Procédé selon l'une quelconque des revendications précédentes, qui comprend en outre l'isolement et le clonage de la cellule produisant la protéine.
